# EUROPEAN PATENT APPLICATION

(11) **EP 4 837 905 A1**
(43) Date of publication of application: **11.11.2026**
(21) Application number: 24914556.6
(22) Date of filing: 03.01.2024
(51) Int. Cl.: C07D 403/10, C12N 5/00, C07D 231/56, C07D 413/10, C07D 401/10, A61K 31/497, A61K 31/4439, A61K 31/416, A61K 31/5377, A61K 31/422, A61P 35/00

(54) **BIPHENYL DERIVATIVE, PHARMACEUTICAL COMPOSITION AND USE THEREOF**

(71) Applicant: iRegene Therapeutics., Ltd, Chendu, Suchuan 610200 (CN)
(72) Inventor: WEI, Jun, Wuhan, Hubei 430074 (CN); CHEN, Xiping, Wuhan, Hubei 430074 (CN); WEI, Mengya, Wuhan, Hubei 430074 (CN); YAN, Ruorong, Wuhan, Hubei 430074 (CN)
(74) Representative: Metida
(86) International application number: PCT/CN2024/070328
(87) International publication number: WO 2025/145323

(57) **Abstract**

The present application relates to the technical field of medicines, in particular to a pyrazolformyl piperazinone compound, a pharmaceutical composition and the use thereof. The pyrazolformyl piperazinone compound has a structure as shown in formula I, or is a pharmaceutically acceptable salt, ester, amide, solvate, active metabolite, polymorph, isotopic label, isomer or prodrug of the structure as shown in formula I.

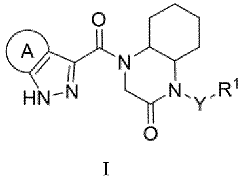

## Description

### Background of the Invention

### Field of the Invention

This application relates to the field of pharmaceutical technology, in particular to a biphenyl derivative, a pharmaceutical composition and the use thereof.

### Description of Related Art

In 2006, Shinya Yamanaka's team proposed a "cocktail" method consisting of four transcription factors: Oct4, Sox2, Klf4, and c-Myc. This method successfully reprograms terminally differentiated skin fibroblasts into pluripotent stem cells, known as induced pluripotent stem cells (iPSCs) (Takahashi K, et al., Cell, 2006, 126(4) pp. 663-676; Takahashi K and Yamanaka S, Cell, 2007, 131(5) pp. 861-872). These stem cells exhibit similar differentiation potential to Embryonic stem cells (ESCs) and can form the three most basic germ layers of human development: ectoderm, mesoderm, and endoderm, and ultimately form various adult cells. The proposal of this method broke through the ethical constraints of using human Embryonic stem cells in medicine and greatly expanded the potential applications of stem cell technology in clinical medicine.

Currently, the widely used reprogramming methods often rely on viruses or other types of vectors to overexpress reprogramming transcription factors represented by Oct4 (Takahashi K, et al., Cell, 2006, 126(4): 663-676; Takahashi K and Yamanaka S, Cell, 2007, 131(5): 861-872; Yu J, et al. Science. 2007; 318: 1917-1920). Such methods pose potential clinical risks in the clinical use of induced pluripotent stem cells (iPSCs), such as the tumorigenic risks brought about by the use of viral vectors. Additionally, the complex GMP production process of vectors introduces complexity to the clinical regulation of induced pluripotent stem cells. Furthermore, the use of vectors in such products results in high costs. Therefore, if selective gene regulation can be achieved using chemical substances to alter the expression state of reprogramming transcription factors, then there will be no need for ectopic expression of "reprogramming genes".

The Wnt/β-catenin pathway is an evolutionarily conserved signaling cascade that is crucial for embryonic development, cellular viability, and tissue regeneration. Furthermore, its deregulation has been linked to tumorigenesis (H. Clevers and R. Nusse, Cell, 2012, 149: 1192-1205). Therefore, the upstream and downstream regulation of the Wnt signaling pathway has become a research hotspot in multiple fields.

Currently, various inhibitors of core proteins in the Wnt signaling pathway have been developed and applied. GSK3 is a Serine/Threonine kinase and a key inhibitor in the Wnt pathway. CHIR99021 is a widely used GSK3 inhibitor, which plays a very significant role in maintaining pluripotency in mammalian embryos (Meek et al., STEM CELLS. 2007, 31, 10, pp. 2104-2115). In addition, combining CHIR99021 with compounds such as valproic acid can enable fibroblasts to become pluripotent stem cells through pure chemical reprogramming (without genetic factors) (Guan et al., Nature. 2022 May; 605(7909):325-331). A recent study has shown that the use of CHIR99021 enables β-catenin downstream of GSK3 to form a complex with Oct4 in a TCF-independent manner, thereby enhancing cellular pluripotency (Fernando F et al., Development, 2013:140, 1171-1183).

The CMGC kinase family is named after the initials of its subfamily members, including cyclin-dependent kinases (CDKs), mitogen-activated protein kinases (MAPKs), glycogen synthase kinases (GSKs), and CDC-like kinases (CLKs). CMGC kinases are highly conserved in organisms (Chowdhury I et al., Cancers (Basel). 2023 Jul 28; 15(15):3838). Taking CLK as an example, it is capable of phosphorylating serine, threonine, and tyrosine residues (Manning G et al., Science. 2002; 298:1912-1934). The structure of CLK is very typical, consisting of N-lobes and C-lobes, which are connected by the "hinge" region of the protein backbone. The structure distributed between the N-region and C-region is the catalytic domain of β- chains and α-helices (Bullock A et al., Structure. 2009; 17:352-362; Keri G et al., Curr. Signal Transduct. Ther. 2006; 1:67-95). CLK further modulates the Wnt signaling pathway by regulating alternative splicing of key Wnt-related genes (Tam B.Y et al., Cancer Lett. 2019: S0304383519304732). Typical representatives of CLK kinases include CLK3 and CLK4. Another subfamily member of CMGC, mitogen-activated protein kinase (MAPK), is a group of serine-threonine protein kinases that can be activated by different extracellular stimuli, such as cytokines, neurotransmitters, hormones, cellular stress, and cell adhesion. Its typical representatives include JNK1 kinase (c-Jun N-terminal kinase 1).

The STE kinase family is involved in the regulation of MAP kinases and CMGC kinase families, playing a central role in the transduction of various extracellular and intracellular signals. For example, the STE20 kinase family member hPAK1 regulates the JNK MAP kinase pathway through GTPase (Brown JL et al., Curr Biol. 1996 May 1; 6(5):598-605). Another STE kinase family member TNIK (Traf2 and Nck-interacting kinase), is also one of the regulatory components of the β-catenin-TCF4 transcription complex. TNIK can promote the activation of downstream target genes of the Wnt signaling pathway through direct interactions with β-catenin and TCF4, playing an important role in cytoskeleton formation and development (Fu et al., JBC, 1999, (274): 30729-30737; Mahmoudi et al., EMBO, 2009, (28): 3329-3340). The TNIK inhibitor NCB-0846 can negatively regulate the TGF-β/SMAD signaling pathway by downregulating the expression of TGFBRI, thereby inhibiting the epithelial-mesenchymal transition process of tumors (Sugano et al., Br J Cancer, 2021, (124): 228-236).

In addition to the key proteins in the Wnt pathway, various kinases upstream and downstream of the Wnt pathway have also been proven to be important regulators of Wnt signaling, such as tyrosine kinases**.** Tyrosine kinases are enzymes that catalyze the transfer of phosphate groups from ATP to tyrosine residues on proteins in cells, thereby regulating the "on" and "off" of signaling pathways in cells. Receptor tyrosine kinases (RTKs) are a type of tyrosine kinases that are activated by the binding of ligands to their extracellular domains **(**Hanks SK et al., Science. 1988 Jul 1; 241(4861):42-52**)**. PDGFRs are one of the core members of the receptor tyrosine kinase (RTKs) family, comprising two isoforms: PDGFRα and PDGFRβ (Guérit et al., Cellular and Molecular Life Sciences, 2021, 78:3867-3881). PDGFRα can also promote early development of multiple cell lineages by inhibiting the Wnt9a/β-catenin signaling pathway (Bartoletti et al., Developmental Biology, 2020, (1):36-46; Sun et al., Cell Stem Cell, 2020 (26):707-721). SU5402 is an inhibitor that simultaneously acts on the FGF, VEGF, and PDGF signaling pathways. SU5402 regulates Wnt signaling by acting on platelet-derived growth factor receptors (PDGFRs).

It is worth noting that although the upstream and downstream pathways of the aforementioned Wnt pathway can exert certain influences on the Wnt pathway, including the GSK3 inhibitor CHIR99021, the PDGF signaling pathway inhibitor SU5402, and the TNIK inhibitor NCB-0846, all of which can regulate certain physiological changes; However, they cannot simultaneously inhibit multiple upstream kinases of the Wnt pathway, nor can they increase the expression of the reprogramming core gene Oct4 during somatic cell reprogramming, thereby further affecting other reprogramming genes and increasing the probability of somatic cell reprogramming.

### Summary of the Invention

Based on this, it is necessary to provide a biphenyl derivative and a pharmaceutical composition containing it. The derivative is designed based on multiple structurally similar Wnt upstream kinases, capable of regulating the pathway through at least one Wnt upstream pathway, and can increase the expression of reprogramming core genes Oct4, Lin28A, and c-Myc in its independent presence. Therefore, it can be well applied to cell reprogramming.

In a first aspect, the present application provides a biphenyl derivative, which has a structure represented by Formula I, or a pharmaceutically acceptable salt, ester, amide, solvate, active metabolite, polymorphic form, isotopic label, isomer, or prodrug thereof;

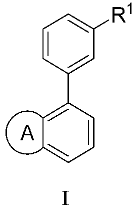

Wherein the A ring is a five-membered ring that is substituted with methyl or amino, or is unsubstituted, and contains one or two nitrogen atoms among its ring atoms;
R¹ is a hydrogen bond donor or acceptor, and its structure contains one or more of amino, imino, hydroxyl, and ether linkage.

In a second aspect, the present application provides a pharmaceutical composition comprising the aforementioned biphenyl derivative and at least one pharmaceutically acceptable carrier.

In a third aspect, the present application provides the use of the aforementioned biphenyl derivative or pharmaceutical composition in cell reprogramming.

In a fourth aspect, the present application provides a method for cell reprogramming, comprising the following steps:
Contacting the cells with the aforementioned biphenyl derivative or pharmaceutical composition.

In a fifth aspect, the present application provides the use of the aforementioned biphenyl derivative or pharmaceutical composition in the inhibition of kinases, wherein the kinases comprise one or more of the TK kinase family, the STE kinase family, and the CMGC kinase family.

In a sixth aspect, the present application provides a method for inhibiting kinases, comprising the following steps:
Contacting the kinase with the aforementioned biphenyl derivative or pharmaceutical composition;
Wherein the kinase comprises one or more of the TK kinase family, STE kinase family, and CMGC kinase family.

### Brief Description of the Figures

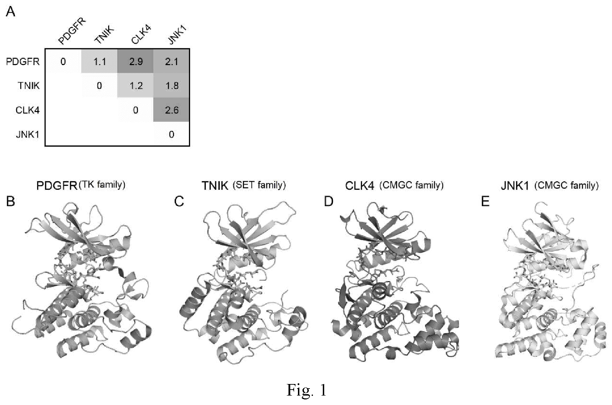

Fig. 1 shows the comparative analysis of the three-dimensional protein structures of PDGFR, TNIK, CLK4, and JNK1. Fig. 1A shows a heatmap displaying the root mean square deviation (RMSD) of protein structures when compared pairwise. The numbers in the heatmap represent the RMSD values for the pairwise structure comparisons. Figs. 1B, 1C, 1D, and 1E show the cartoon models of the three-dimensional structures of PDGFR (PDB ID: 5GRN) (Liang L et al., Biochem Biophys Res Commun. 2016 Sep 2; 477(4): 667-672), TNIK (PDB ID: 5AX9) (Masuda M et al., Nat Commun. 2016 Aug 26; 7: 12586), CLK4 (PDB ID: 6FYV) (Kallen J et al., ChemMedChem. 2018 Sep 19; 13(18): 1997-2007), and JNK1 (PDB ID: 3ELJ) (Chamberlain SD et al., Bioorg Med Chem Lett. 2009 Jan 15; 19(2): 360-4), respectively. The key site of the active center of the protein (Fig. 1B) are shown as stick models.

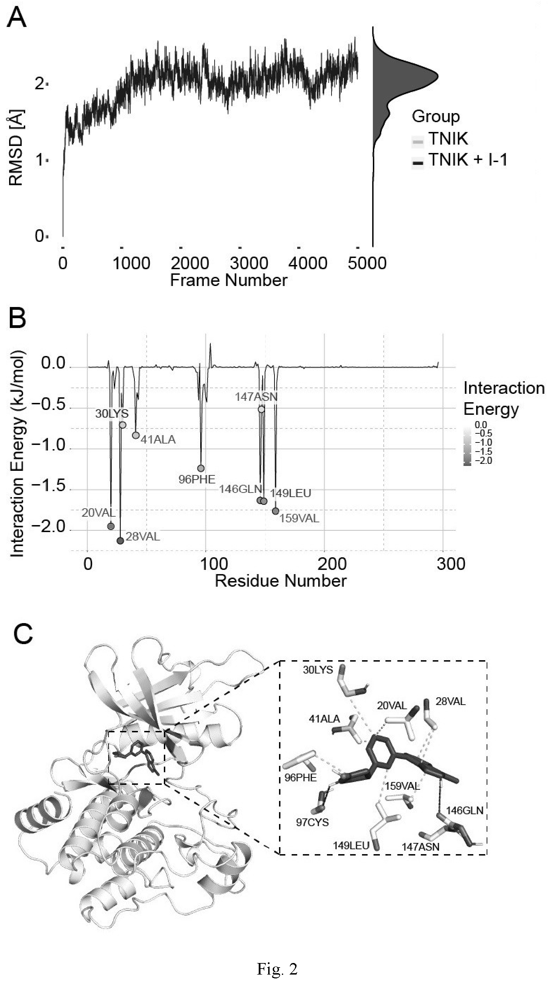

Fig. 2 shows the binding ability and chemical bond details of the biphenyl derivative I-1 with TNIK through dynamics simulation. Panel A shows the variation graph of the root mean square deviation (RMSD) of the complex of TNIK and I-1 with simulation time during the simulation process, indicating that TNIK can bind to I-1 and the resulting complex structure is in a stable binding state. Panel B shows an energy decomposition diagram of the interactions between each amino acid site of TNIK and I-1, with the x-axis representing the amino acid sequence of TNIK and the y-axis indicating the energy values contributed by each amino acid site to ligand binding. The names of amino acid sites with a ligand binding energy contribution less than -0.5 kJ/mol have been marked. Panel C displays the stabilized complex structure of TNIK and I-1 from molecular dynamics simulation. with proteins represented in a cartoon model. The colour of each site indicates its energy contribution to ligand binding, while small molecules and the main or side chains of the residues interacting with them are depicted as stick models. Hydrogen bonding interactions are represented by long dashed lines, and hydrophobic interactions are depicted by short dashed lines. This result indicates that the active catalytic center of the kinase target bound by TNIK with I-1 and the chemical basis of their interaction are clear, that is, the basis for the formation of chemical bonds is consistent.

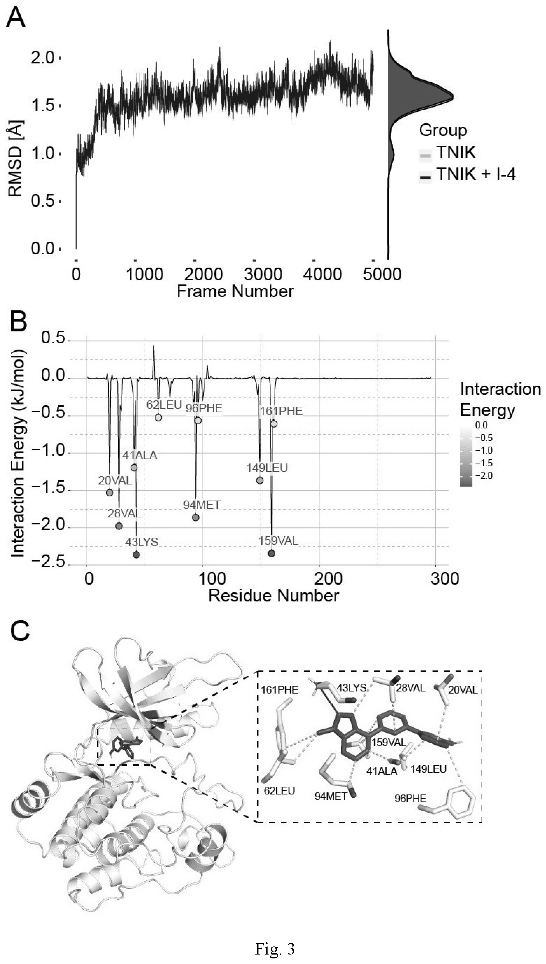

Fig. 3 shows the binding ability and chemical bond details of the biphenyl derivative I-4 with TNIK through kinetic simulation. Panel A shows the variation graph of the root mean square deviation (RMSD) of the complex of TNIK and I-4 with simulation time during the simulation process, indicating that TNIK can bind to I-4 and the resulting complex structure is in a stable binding state. Panel B shows an energy decomposition diagram of the interactions between each amino acid site of TNIK and I-4, with the x-axis representing the amino acid sequence of TNIK and the y-axis indicating the energy values contributed by each amino acid site to ligand binding. The names of amino acid sites with a ligand binding energy contribution less than -0.5 kJ/mol have been marked. Panel C displays the stabilized complex structure of TNIK and I-4 from molecular dynamics simulation, with proteins represented in a cartoon model. The colour of each site indicates its energy contribution to ligand binding, while small molecules and the main or side chains of the residues interacting with it are depicted as stick models. Hydrogen bonding interactions are represented by long dashed lines, and hydrophobic interactions are depicted by short dashed lines. This result indicates that the active catalytic center of the kinase target bound by TNIK and I-4 and the chemical basis of their interaction are clear, that is, the basis for the formation of chemical bonds is consistent.

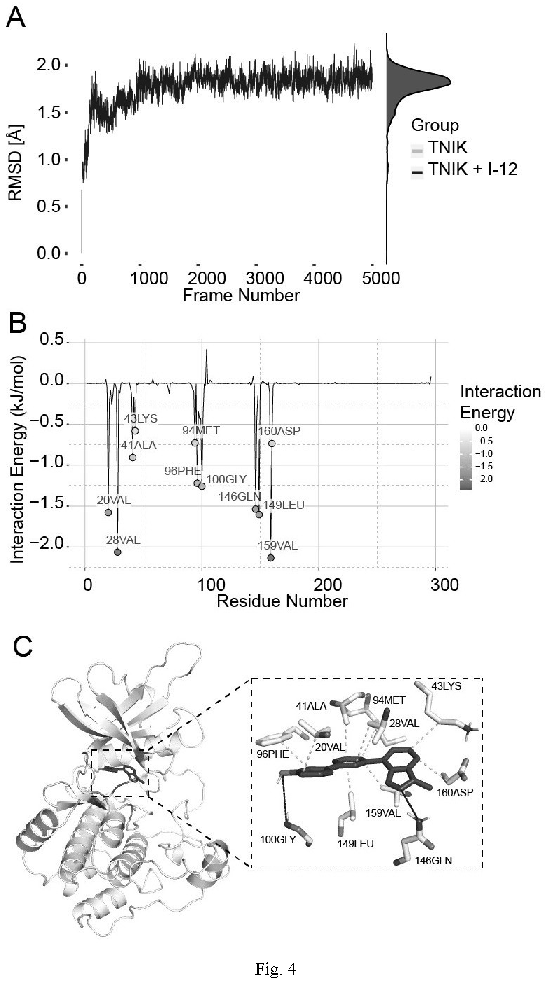

Fig. 4 shows the binding ability and chemical bond details of the biphenyl derivative I-12 with TNIK through kinetic simulation. Panel A shows the variation graph of the root mean square deviation (RMSD) of the complex of TNIK and I-12 with simulation time during the simulation process, indicating that TNIK can bind to I-12 and the resulting complex structure is in a stable binding state. Panel B shows an energy decomposition diagram of the interactions between each amino acid site of TNIK and I-12, with the x-axis representing the amino acid sequence of TNIK and the y-axis indicating the energy values contributed by each amino acid site to ligand binding. The names of amino acid sites with a ligand binding energy contribution less than -0.5 kJ/mol have been marked. Panel C displays the stabilized complex structure of TNIK and I-12 from molecular dynamics simulation, with proteins represented in a cartoon model. The colour of each site indicates its energy contribution to ligand binding, while small molecules and the main or side chains of the residues interacting with it are depicted as stick models. Hydrogen bonding interactions are represented by long dashed lines, and hydrophobic interactions are depicted by short dashed lines. This result indicates that the active catalytic center of the kinase target bound by TNIK and I-12 and the chemical basis of their interaction are clear, that is, the basis for the formation of chemical bonds is consistent.

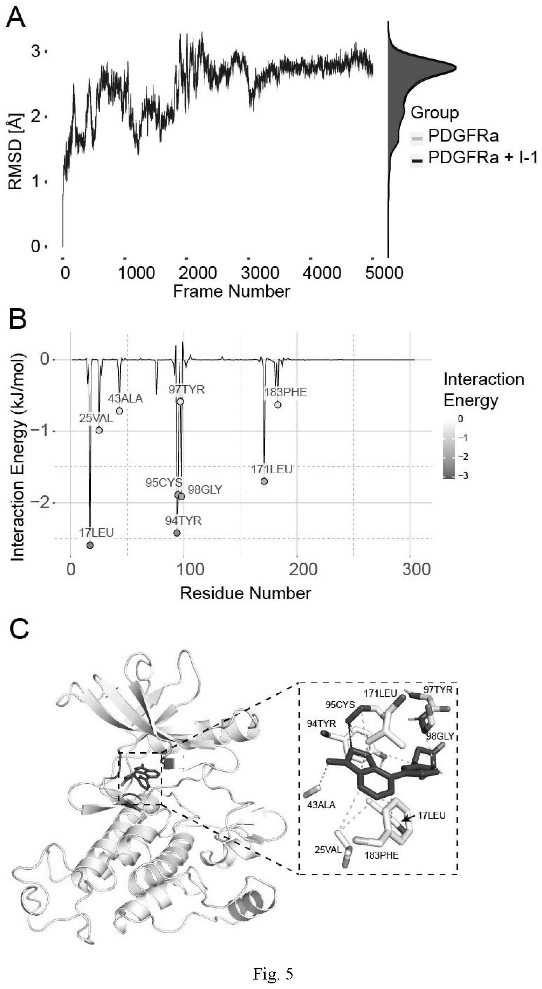

Fig. 5 shows the binding ability and chemical bond details of the biphenyl derivative I-1 with PDGFRα through dynamics simulation. Panel A shows the variation graph of the root mean square deviation (RMSD) of the complex of PDGFRα and I-1 with simulation time during the simulation process, indicating that PDGFRα can bind to I-1 and the resulting complex structure is in a stable binding state. Panel B shows an energy decomposition diagram of the interactions between each amino acid site of PDGFRα and I-1, with the x-axis representing the amino acid sequence of PDGFRα and the y-axis indicating the energy values contributed by each amino acid site to ligand binding. The names of amino acid sites with a ligand binding energy contribution less than -0.5 kJ/mol have been marked. Panel C displays the stabilized complex structure of PDGFRα and I-1 from molecular dynamics simulation, with proteins represented in a cartoon model. The colour of each site indicates its energy contribution to ligand binding, while small molecules and the main or side chains of the residues interacting with it are depicted as stick models. Hydrogen bonding interactions are represented by long dashed lines, and hydrophobic interactions are depicted by short dashed lines. This result indicates that the active catalytic center of the kinase target bound by PDGFRα and I-1 and the chemical basis of their interaction are clear, that is, the basis for the formation of chemical bonds is consistent.

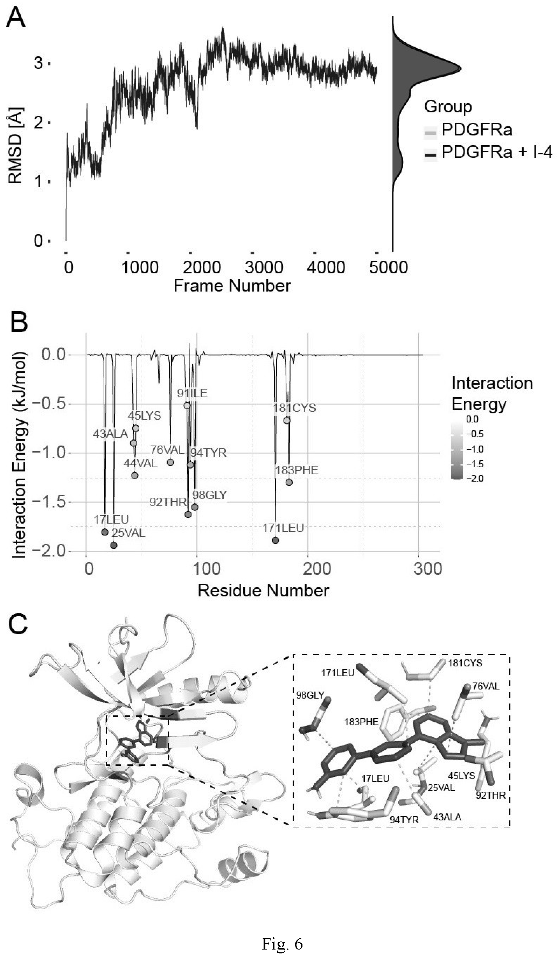

Fig. 6 shows the binding ability and chemical bond details of the biphenyl derivative I-4 with PDGFRα through dynamics simulation. Panel A shows the variation graph of the root mean square deviation (RMSD) of the complex of PDGFRα and I-4 with simulation time during the simulation process, indicating that PDGFRα can bind to I-4 and the resulting complex structure is in a stable binding state. Panel B shows an energy decomposition diagram of the interactions between each amino acid site of PDGFRα and I-4, with the x-axis representing the amino acid sequence of PDGFRα and the y-axis indicating the energy values contributed by each amino acid site to ligand binding. The names of amino acid sites with a ligand binding energy contribution less than -0.5 kJ/mol have been marked. Panel C displays the stabilized complex structure of PDGFRα and I-4 from molecular dynamics simulation, with proteins represented in a cartoon model. The colour of each site indicates its energy contribution to ligand binding, while small molecules and the main or side chains of the residues interacting with it are depicted as stick models. Hydrogen bonding interactions are represented by long dashed lines, and hydrophobic interactions are depicted by short dashed lines. This result indicates that the active catalytic center of the kinase target bound by PDGFRα and I-4 and the chemical basis of their interaction are clear, that is, the basis for the formation of chemical bonds is consistent.

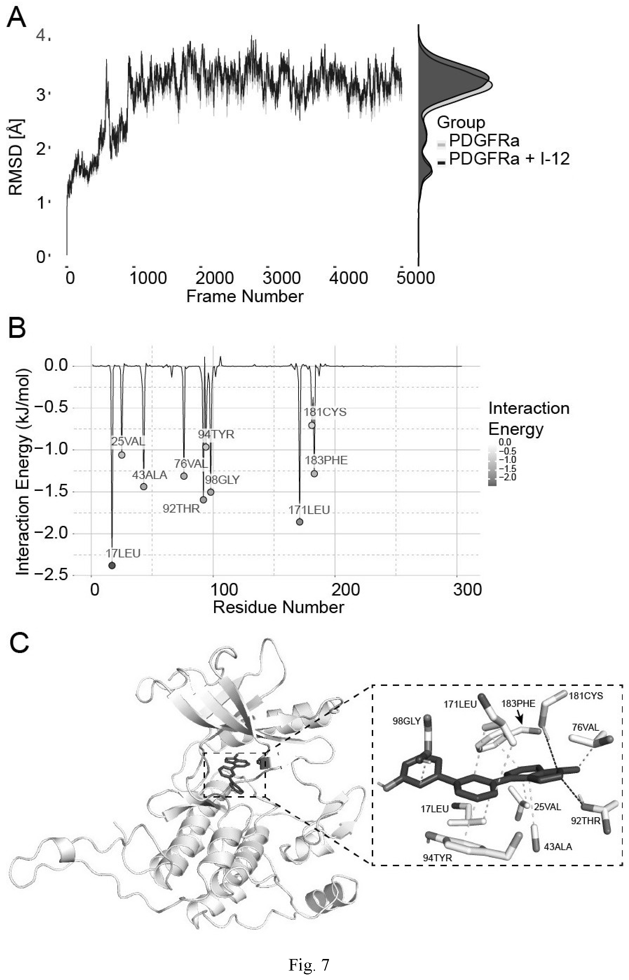

Fig. 7 shows the binding ability and chemical bond details of the biphenyl derivative I-12 with PDGFRα through dynamics simulation. Panel A shows the variation graph of the root mean square deviation (RMSD) of the complex of PDGFRα and I-12 with simulation time during the simulation process, indicating that PDGFRα can bind to I-12 and the resulting complex structure is in a stable binding state. Panel B shows an energy decomposition diagram of the interactions between each amino acid site of PDGFRα and I-12, with the x-axis representing the amino acid sequence of PDGFRα and the y-axis indicating the energy values contributed by each amino acid site to ligand binding. The names of amino acid sites with a ligand binding energy contribution less than -0.5 kJ/mol have been marked. Panel C displays the stabilized complex structure of PDGFRα and I-12 from molecular dynamics simulation, with proteins represented in a cartoon model. The colour of each site indicates its energy contribution to ligand binding, while small molecules and the main or side chains of the residues interacting with it are depicted as stick models. Hydrogen bonding interactions are represented by long dashed lines, and hydrophobic interactions are depicted by short dashed lines. This result indicates that the active catalytic center of the kinase target bound by PDGFRα and I-12 and the chemical basis of their interaction are clear, that is, the basis for the formation of chemical bonds is consistent.

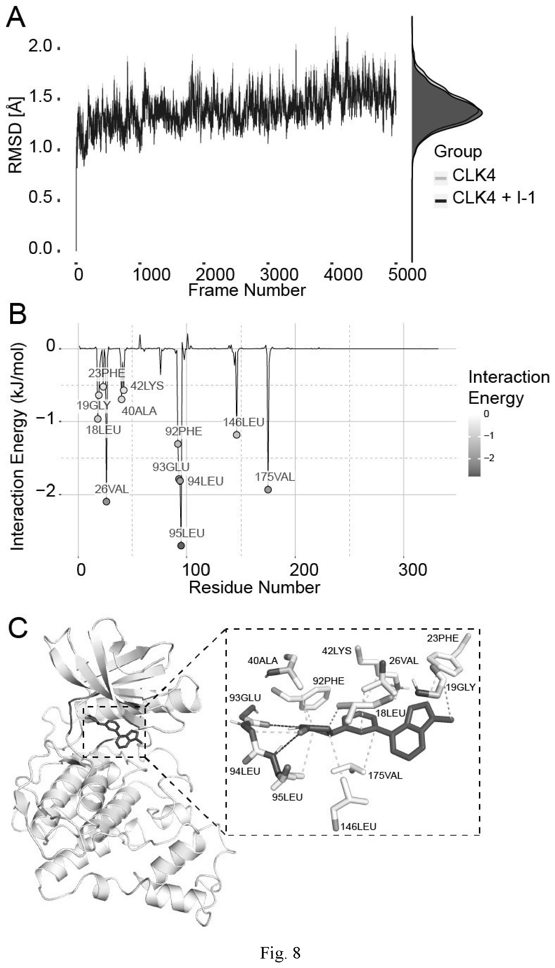

Fig. 8 shows the binding ability and chemical bond details of the biphenyl derivative I-1 with CLK4 through dynamics simulation. Panel A shows the variation graph of the root mean square deviation (RMSD) of the complex of CLK4 and I-1 with simulation time during the simulation process, indicating that CLK4 can bind to I-1 and the resulting complex structure is in a stable binding state. Panel B shows an energy decomposition diagram of the interactions between each amino acid site of CLK4 and I-1, with the x-axis representing the amino acid sequence of CLK4 and the y-axis indicating the energy values contributed by each amino acid site to ligand binding. The names of amino acid sites with a ligand binding energy contribution less than -0.5 kJ/mol have been marked. Panel C displays the stabilized complex structure of CLK4 and I-1 from molecular dynamics simulation, with proteins represented in a cartoon model. The colour of each site indicates its energy contribution to ligand binding, while small molecules and the main or side chains of the residues interacting with it are depicted as stick models. Hydrogen bonding interactions are represented by long dashed lines, and hydrophobic interactions are depicted by short dashed lines. This result indicates that the active catalytic center of the kinase target bound by CLK4 and I-1 and the chemical basis of their interaction are clear, that is, the basis for the formation of chemical bonds is consistent.

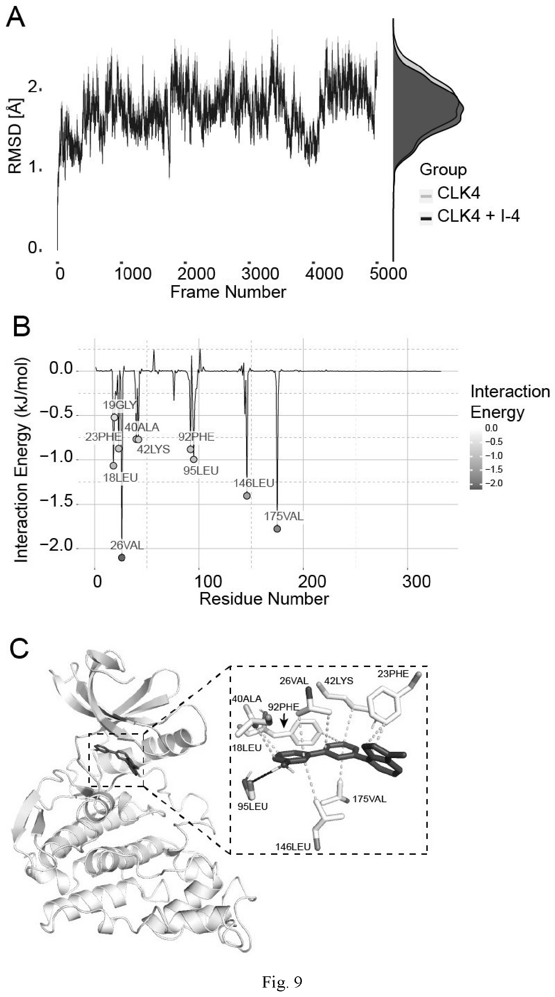

Fig. 9 shows the binding ability and chemical bond details of the biphenyl derivative I-4 with CLK4 through dynamics simulation. Panel A shows the variation graph of the root mean square deviation (RMSD) of the complex of CLK4 and I-4 with simulation time during the simulation process, indicating that CLK4 can bind to I-4 and the resulting complex structure is in a stable binding state. Panel B shows an energy decomposition diagram of the interactions between each amino acid site of CLK4 and I-4, with the x-axis representing the amino acid sequence of CLK4 and the y-axis indicating the energy values contributed by each amino acid site to ligand binding. The names of amino acid sites with a ligand binding energy contribution less than -0.5 kJ/mol have been marked. Panel C displays the stabilized complex structure of CLK4 and I-4 from molecular dynamics simulation, with proteins represented in a cartoon model. The colour of each site indicates its energy contribution to ligand binding, while small molecules and the main or side chains of the residues interacting with it are depicted as stick models. Hydrogen bonding interactions are represented by long dashed lines, and hydrophobic interactions are depicted by short dashed lines. This result indicates that the active catalytic center of the kinase target bound by CLK4 and I-4 and the chemical basis of their interaction are clear, that is, the basis for the formation of chemical bonds is consistent.

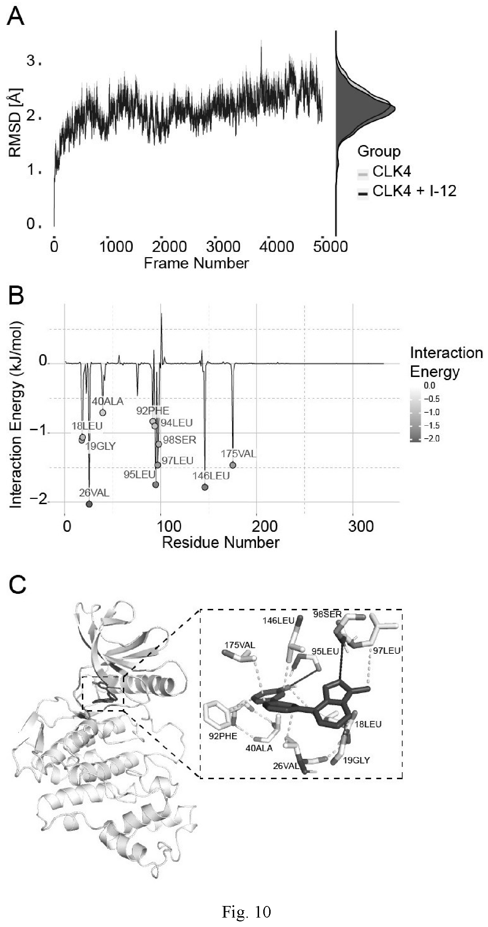

Fig. 10 shows the binding ability and chemical bond details of the biphenyl derivative I-12 with CLK4 through dynamics simulation. Panel A shows the variation graph of the root mean square deviation (RMSD) of the complex of CLK4 and I-12 with simulation time during the simulation process, indicating that CLK4 can bind to I-12 and the resulting complex structure is in a stable binding state. Panel B shows an energy decomposition diagram of the interactions between each amino acid site of CLK4 and I-12, with the x-axis representing the amino acid sequence of CLK4 and the y-axis indicating the energy values contributed by each amino acid site to ligand binding. The names of amino acid sites with a ligand binding energy contribution less than -0.5 kJ/mol have been marked. Panel C displays the stabilized complex structure of CLK4 and I-12 from molecular dynamics simulation, with proteins represented in a cartoon model. The colour of each site indicates its energy contribution to ligand binding, while small molecules and the main or side chains of the residues interacting with it are depicted as stick models. Hydrogen bonding interactions are represented by long dashed lines, and hydrophobic interactions are depicted by short dashed lines. This result indicates that the active catalytic center of the kinase target bound by CLK4 and I-12 and the chemical basis of their interaction are clear, that is, the basis for the formation of chemical bonds is consistent.

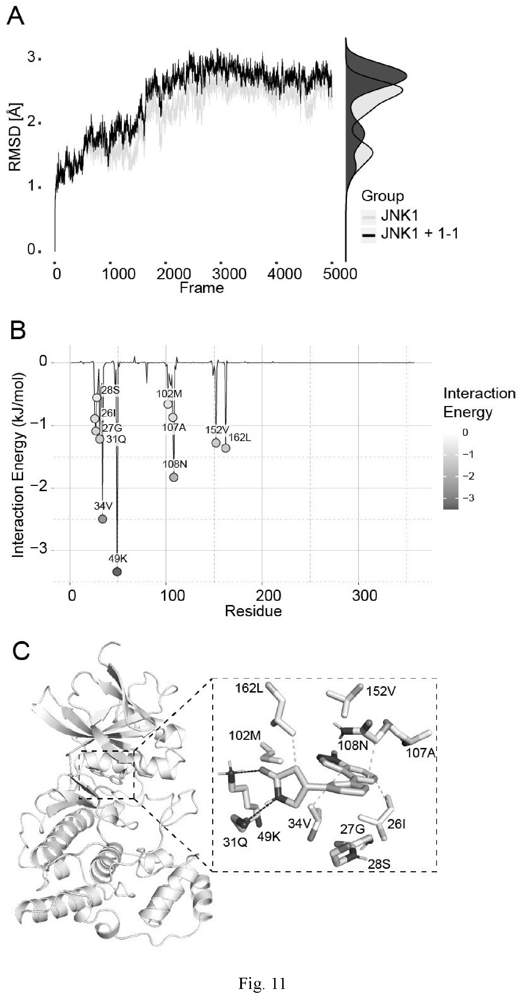

Fig. 11 shows the binding ability and chemical bond details of the biphenyl derivative I-1 with JNK1 through dynamics simulation. Panel A shows the variation graph of the root mean square deviation (RMSD) of the complex of JNK1 and I-1 with simulation time during the simulation process, indicating that JNK1 can bind to I-1 and the resulting complex structure is in a stable binding state. Panel B shows an energy decomposition diagram of the interactions between each amino acid site of JNK1 and I-1, with the x-axis representing the amino acid sequence of JNK1 and the y-axis indicating the energy values contributed by each amino acid site to ligand binding. The names of amino acid sites with a ligand binding energy contribution less than -0.5 kJ/mol have been marked. Panel C displays the stabilized complex structure of JNK1 and I-1 from molecular dynamics simulation, with proteins represented in a cartoon model. The colour of each site indicates its energy contribution to ligand binding, while small molecules and the main or side chains of the residues interacting with it are depicted as stick models. Hydrogen bonding interactions are represented by long dashed lines, and hydrophobic interactions are depicted by short dashed lines. This result indicates that the active catalytic center of the kinase target bound by JNK1 and I-1 and the chemical basis of their interaction are clear, that is, the basis for the formation of chemical bonds is consistent.

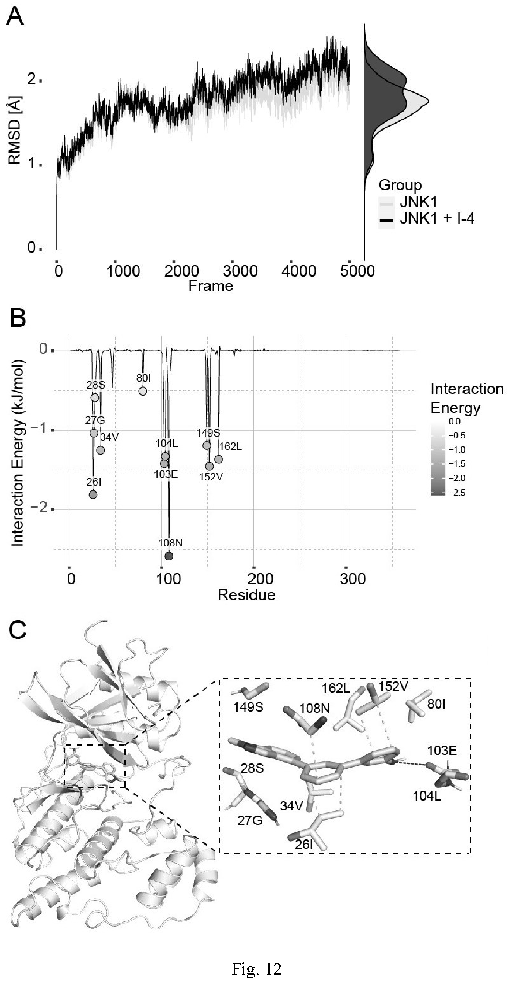

Fig. 12 shows the binding ability and chemical bond details of the biphenyl derivative I-4 with JNK1 through dynamics simulation. Panel A shows the variation graph of the root mean square deviation (RMSD) of the complex of JNK1 and I-4 with simulation time during the simulation process, indicating that JNK1 can bind to I-4 and the resulting complex structure is in a stable binding state. Panel B shows an energy decomposition diagram of the interactions between each amino acid site of JNK1 and I-4, with the x-axis representing the amino acid sequence of JNK1 and the y-axis indicating the energy values contributed by each amino acid site to ligand binding. The names of amino acid sites with a ligand binding energy contribution less than -0.5 kJ/mol have been marked. Panel C displays the stabilized complex structure of JNK1 and I-4 from molecular dynamics simulation, with proteins represented in a cartoon model. The colour of each site indicates its energy contribution to ligand binding, while small molecules and the main or side chains of the residues interacting with it are depicted as stick models. Hydrogen bonding interactions are represented by long dashed lines, and hydrophobic interactions are depicted by short dashed lines. This result indicates that the active catalytic center of the kinase target bound by JNK1 and I-4 and the chemical basis of their interaction are clear, that is, the basis for the formation of chemical bonds is consistent.

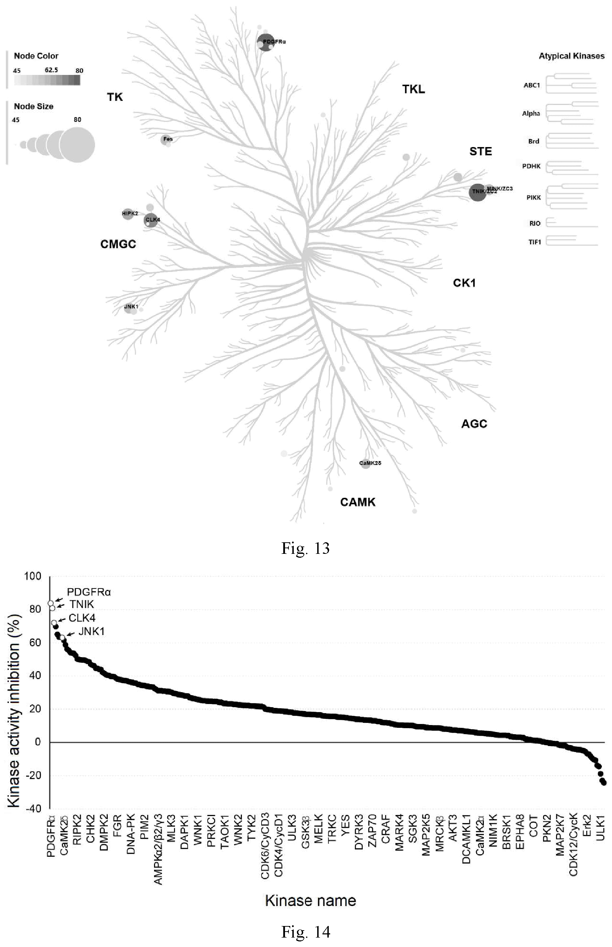

Fig. 13 shows the visual analysis of the activity test results of biphenyl derivative I-1 in the kinase group range. In this analysis, each node represents a kinase, and the size and color intensity of the node indicate the inhibitory effect of I-1 on each kinase. The results demonstrate that I-1 has actual binding effects on each target kinase. The kinase group evolutionary tree references Cell Signaling Technology: www.cellsignal.com.
Fig. 14 shows the kinase targets affected by biphenyl derivatives based on kinase activity screening analysis. It displays the effect analysis of biphenyl derivative I-1 on the enzymatic activity of 330 kinases (the name of each kinase is displayed every 10 kinases along the x-axis).

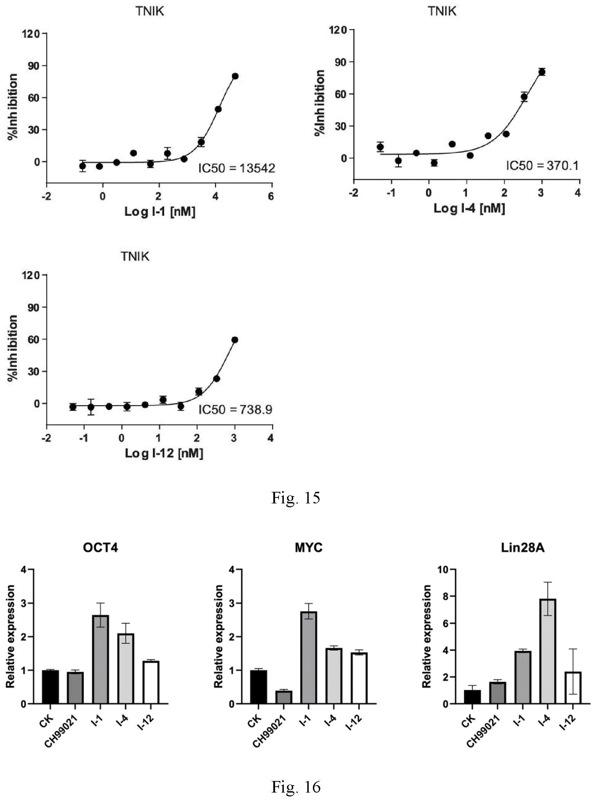

Fig. 15 shows the IC₅₀ results of biphenyl derivatives on against the target kinase. The data indicate that the biphenyl derivatives exhibit binding affinity to the target kinase and demonstrate significant antagonistic activity against it.
Fig. 16 shows that biphenyl derivatives have a significant enhancing effect on the expression of reprogramming core genes Oct4, c-Myc, and Lin28A. Compared to the blank control (CK) without the addition of compounds, biphenyl derivatives can simultaneously enhance the expression of this group of reprogramming core genes, Oct4, c-Myc, and Lin28A, in their independent presence.

### Detailed Description of the Invention

For the purpose of facilitating the understanding of the present application, the following provides a more detailed description of the present application with reference to the accompanying drawings. The drawings illustrate preferred embodiments of the present application. However, the present application may be implemented in many different forms and is not limited to the embodiments described herein. Rather, the purpose of providing these embodiments is to enable a more thorough and comprehensive understanding of the disclosure of the present application.

Unless otherwise defined, all technical and scientific terms used herein have the same meanings as those commonly understood by persons skilled in the art to which the present application pertains. The terminology used in the specification of the present application is solely for the purpose of describing particular embodiments and is not intended to limit the present application. The term "and/or" as used herein includes any and all combinations of one or more of the listed items.

In the present application, an open-ended description of technical features includes both a closed-ended technical solution composed of the listed features and an open-ended technical solution comprising the listed features.

In the present application, numerical ranges are considered continuous and include the minimum and maximum values of the range, as well as any value therebetween, unless otherwise specified. Furthermore, when the range refers to integers, it includes every integer between the minimum and maximum values. In addition, when multiple ranges are described for a feature or property, the ranges may be combined. In other words, unless otherwise indicated, all ranges disclosed herein are understood to include any and all sub-ranges therein.

Percentages disclosed in the present application, unless otherwise specified, refer to weight percent for solid-liquid and solid-solid mixtures, and volume percent for liquid-liquid mixtures.

Percentage concentrations disclosed herein, unless otherwise specified, refer to the final concentration, which is the proportion of the added component in the system after addition.

Temperature parameters disclosed herein, unless otherwise specified, allow for both constant-temperature treatment and treatment within a certain temperature range. Constant-temperature treatment permits fluctuations within the precision range of the instrument.

### Terminology Definitions

The "ring atoms" mentioned in this application refer to the atoms involved in the formation of the A ring skeleton, excluding the substituents on the A ring. For example, if the A ring has the structure shown in formula A-1-1, its ring atoms are the two carbon atoms at the attachment sites, two nitrogen atoms, and the carbon atom forming the carbon-nitrogen double bond. The carbon atom in the substituent methyl on the nitrogen atom does not belong to the ring atoms.

"Hydrogen bond donor" refers to a group containing a hydrogen atom bonded to an atom with strong electronegativity and small radius, such as O, N, or F, examples include groups containing NH₂ or OH. "Hydrogen bond acceptor" refers to a group containing an atom with strong electronegativity and small radius, such as O, N, or F. In this application, R¹ can serve as either a hydrogen bond donor or acceptor, inhibiting kinases by forming hydrogen bonds with protein structures.

The term "prodrug" refers to any compound that, upon administration to a biological system, undergoes spontaneous chemical reaction, enzymatic transformation, photolysis, and/or metabolic chemical reaction to produce the active drug, i.e., the active ingredient. Prodrugs are thus covalently modified analogs or latent forms of therapeutically active compounds. Suitable examples include, but are not limited to: carboxylic esters, carbonates, phosphate esters, nitrate esters, sulfate esters, sulfonates, sulfamates, amino compounds, carbamates, azo compounds, phosphoramides, glycosides, ethers, acetal derivatives, and the like.

The term "pharmaceutically acceptable" refers to ligands, materials, compositions, and/or dosage forms that are suitable for administration to a patient within the bounds of reasonable medical judgment and having a reasonable benefit/risk ratio.

The term "pharmaceutically acceptable carrier" refers to pharmaceutically acceptable materials, compositions, or vehicles, such as liquid or solid fillers, diluents, excipients, solvents, or encapsulating materials. As used herein, the term "pharmaceutically acceptable carrier" includes buffers, sterile water for injection, solvents, dispersing media, coatings, antibacterial and antifungal agents, isotonic agents, absorption retardants, and the like, which are compatible with the administration of the drug. Each carrier must be "pharmaceutically acceptable" in the sense of being compatible with other ingredients of the formulation and non-toxic to the patient. Suitable examples include, but are not limited to: (1) sugars such as lactose, glucose, and sucrose; (2) starches such as corn starch, potato starch, and substituted or unsubstituted β-cyclodextrins; (3) cellulose and derivatives such as sodium carboxymethylcellulose, ethylcellulose, and cellulose acetate; (4) powdered tragacanth; (5) malt; (6) gelatin; (7) talc; (8) excipients such as cocoa butter and suppository waxes; (9) oils such as peanut oil, cottonseed oil, safflower oil, sesame oil, olive oil, corn oil, and soybean oil; (10) glycols such as propylene glycol; (11) polyols such as glycerol, sorbitol, mannitol, and polyethylene glycol; (12) esters such as ethyl oleate and ethyl laurate; (13) agar; (14) buffers such as magnesium hydroxide and aluminum hydroxide; (15) alginates; (16) pyrogen-free water; (17) isotonic saline; (18) Ringer's solution; (19) ethanol; (20) phosphate-buffered saline; and (21) other non-toxic compatible substances used in pharmaceutical formulations.

The term "pharmaceutically acceptable salt" refers to any salt of the compounds disclosed herein with acids or bases suitable for use as a medicament. Pharmaceutically acceptable salts include inorganic and organic salts. One type of salt is a salt of the disclosed compound with an acid. Suitable acids include, but are not limited to: inorganic acids such as hydrochloric acid, hydrobromic acid, hydrofluoric acid, sulfuric acid, nitric acid, and phosphoric acid; organic acids such as formic acid, acetic acid, trifluoroacetic acid, propionic acid, oxalic acid, malonic acid, succinic acid, fumaric acid, maleic acid, lactic acid, malic acid, tartaric acid, citric acid, benzoic acid, methanesulfonic acid, ethanesulfonic acid, p-toluenesulfonic acid, benzenesulfonic acid, naphthalenesulfonic acid; and amino acids such as proline, phenylalanine, aspartic acid, glutamic acid. Another type of salt is a salt of the disclosed compound with a base. Suitable bases include, but are not limited to: alkali metal salts (e.g., sodium or potassium), alkaline earth metal salts (e.g., magnesium or calcium), ammonium salts (e.g., lower alkanol ammonium salts and other pharmaceutically acceptable amine salts) such as methylamine, ethylamine, propylamine, dimethylamine, trimethylamine, diethylamine, triethylamine, tert-butylamine, ethylenediamine, hydroxyethylamine, dihydroxyethylamine, tri(hydroxyethyl)amine, and amine salts derived from morpholine, piperazine, or lysine.

The term "pharmaceutically acceptable ester or amide" refers to any ester or amide of the disclosed compounds suitable for use as a medicament. Pharmaceutically acceptable esters include organic and inorganic esters.

In the case where the compound of Formula I contains a hydroxyl group, pharmaceutically acceptable esters can be prepared by conventional condensation reactions with carboxylic acids, acyl chlorides, acid anhydrides, or their active derivatives.

The aforementioned esters may include, for example, C₁-C₆ alkyl esters such as methyl, ethyl, propyl, isopropyl, butyl, sec-butyl, tert-butyl, pentyl, and hexyl esters; C₃-C₆ cycloalkyl esters such as cyclopentyl and cyclohexyl esters; C₆-C₁₀ aryl esters such as phenyl and naphthyl esters; C₆-C₁₀ aryl-C₁-C₆ alkyl esters such as benzyl, phenethyl, α-methylbenzyl, 3-phenylpropyl, 4-phenylbutyl, 6-phenylhexyl, diphenylmethyl, and triphenylmethyl esters; or hydrolyzable esters in vivo such as (5-methyl-2-oxo-1,3-dioxolen-4-yl)methyl ester, neopentyl ester, benzofuranone ester, [(isopropoxycarbonyl)oxy]methyl ester, [(cyclohexyloxycarbonyl)oxy]methyl ester, and 1-[(cyclohexyloxycarbonyl)oxy]ethyl ester.

The aforementioned amides may include, for example, primary amides (-CONH₂); mono-C₁-C₆ alkyl or mono-C₃-C₆ cycloalkyl amides such as N-methylamide, N-ethylamide, N-propylamide, N-isopropylamide, N-butylamide, N-sec-butylamide, N-tert-butylamide, N-pentylamide, N-hexylamide, N-cyclopropylamide, N-cyclopentylamide, and N-cyclohexylamide; or di-C₁-C₆ alkyl, N-C₁-C₆ alkyl-N-C₃-C₆ cycloalkyl, or di-C₃-C₆ cycloalkyl amides such as N,N-dimethylamide, N,N-diethylamide, N,N-dipropylamide, N,N-diisopropylamide, N-methyl-N-ethylamide, N-methyl-N-propylamide, N-methyl-N-butylamide, N-ethyl-N-propylamide, N-ethyl-N-butylamide, N-butyl-N-cyclopentylamide, N-ethyl-N-cyclopropylamide, and N,N-dicyclohexylamide.

The term "solvate" refers to a complex formed between a compound of Formula I and solvent molecules in a defined stoichiometric ratio. The term "hydrate" refers to a complex formed between the compound of the present application and water.

The term "active metabolite" refers to a derivative of the compound that exhibits pharmacological activity upon metabolism of the compound.

The term "polymorph" refers to the compound of the present application existing in different crystalline lattice forms.

The term "isotopically labeled compound" refers to a compound of the present application labeled with one or more isotopes. Isotopes may include, for example, isotopes of H, C, N, O, P, F, and S, such as ²H, ³H, ¹³C, ¹⁴C, ¹⁵N, ¹⁸O, ¹⁷O, ³¹P, ³²P, ³⁵S, ¹⁸F, and ³⁶S.

The term "isomer" refers to compounds having the same molecular formula but differing in the spatial arrangement of atoms. Compounds of the present application may contain asymmetric or chiral centers, double bonds, or other structural elements and may thus include optical isomers, geometric isomers, tautomers, atropisomers, and other stereoisomeric forms. These isomers, including individual stereoisomers and racemates, are encompassed within the scope of the present application. For example, optical isomers can be prepared using chiral resolution, chiral synthesis, chiral reagents, or other conventional techniques to obtain optically active (*R*)- and (*S*)-enantiomers, as well as D and L forms. Non-racemic isomers can also be generated by reaction with a suitable chiral reagent (e.g., a chiral alcohol or Mosher's acid chloride), separated, and converted (e.g., by hydrolysis) to the corresponding single stereoisomer. Separation can also be achieved via chromatographic methods.

"Pharmaceutical compositions" may be prepared by methods well known in the art and can be administered by various routes depending on the need for local or systemic treatment and the target area.

### Modes of Administration

The dosage forms and modes of administration of the compounds of the present application or pharmaceutical compositions thereof are not particularly limited.

Representative modes of administration include, but are not limited to, oral, intra tumoral, rectal, parenteral (intravenous, intramuscular, or subcutaneous) injection, and topical administration.

Solid dosage forms for oral administration include capsules, tablets, pills, powders, and granules. In these solid dosage forms, the active compound can be mixed with at least one conventional inert excipient (or carrier), such as sodium citrate or dicalcium phosphate, or with one or more of the following: (a) fillers or bulking agents, e.g., starch, lactose, sucrose, glucose, mannitol, and silicates; (b) binders, e.g., Hydroxymethylcellulose, alginates, gelatin, polyvinylpyrrolidone, sucrose, and gum Arabic; (c) humectants, e.g., glycerin; (d) disintegrants, e.g., agar, calcium carbonate, potato starch, tapioca starch, alginate, certain composite silicates, and sodium carbonate; (e) retardants, e.g., paraffin; (f) absorption accelerators, e.g., quaternary ammonium compounds; (g) wetting agents, e.g., cetyl alcohol and glyceryl monostearate; (h) adsorbents, e.g., kaolin; and (i) lubricants, e.g., talc, calcium stearate, magnesium stearate, solid polyethylene glycol, sodium lauryl sulfate, or mixtures thereof. Capsules, tablets, and pills may also contain buffering agents. Solid dosage forms such as tablets, sugar-coated pills, capsules, pills, and granules may be prepared with coatings or shells, e.g., enteric coatings or other materials known in the art. Such coatings may include opacifying agents and may allow delayed release of the active compound in a specific region of the gastrointestinal tract. Exemplary encapsulating components include polymers and waxes. If necessary, the active compound may also be formulated as microcapsules with one or more of the excipients described above.

Liquid dosage forms for oral administration include pharmaceutically acceptable emulsions, solutions, suspensions, syrups, or tinctures. In addition to the active compound, liquid dosage forms may contain inert diluents conventionally used in the art, such as water or other solvents, solubilizers, and emulsifiers, e.g., ethanol, isopropanol, ethyl carbonate, ethyl acetate, propylene glycol, 1,3-butanediol, dimethylformamide, and oils, particularly cottonseed oil, peanut oil, corn germ oil, olive oil, castor oil, sesame oil, or mixtures thereof. In addition to these inert diluents, the compositions may also contain adjuvants such as wetting agents, emulsifiers, suspending agents, sweeteners, flavoring agents, and perfumes. Suspensions may contain suspending agents, e.g., ethoxylated isostearyl alcohol, polyoxyethylene sorbitol and dehydrated sorbitol esters, microcrystalline cellulose, aluminum methoxide, agar, or mixtures thereof.

Parenteral compositions may include physiologically acceptable sterile aqueous or non-aqueous solutions, dispersions, suspensions, or emulsions, as well as sterile powders for reconstitution into sterile injectable solutions or dispersions. Suitable aqueous or non-aqueous carriers, diluents, solvents, or excipients include water, ethanol, polyols, and suitable mixtures thereof.

Topical dosage forms include ointments, powders, patches, sprays, and inhalation formulations. They may be prepared by mixing the active ingredient under sterile conditions with a pharmaceutically acceptable carrier and any preservative, buffer, or, if necessary, a propellant.

As used herein, "pharmaceutical" includes any agent, compound, composition, or mixture that provides a physiological and/or pharmacological effect in vivo or in vitro, typically producing a beneficial effect. The range of physiological and/or pharmacological effects produced by the "pharmaceutical" is not particularly limited and may be systemic or localized. The active agent in the "pharmaceutical" is not specifically limited and may be an agent capable of interacting with other substances or an inert substance that does not interact.

In a first aspect, the present application provides a biphenyl derivative, which has a structure represented by Formula I, or a pharmaceutically acceptable salt, ester, amide, solvate, active metabolite, polymorphic form, isotopic label, isomer, or prodrug thereof;

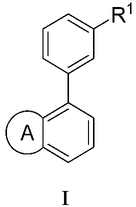

Wherein the A ring is a five-membered ring that is substituted with methyl or amino, or is unsubstituted, and contains one or two nitrogen atoms among its ring atoms;
R¹ is a hydrogen bond donor or acceptor, and its structure contains one or more of amino, imino, hydroxyl, and ether linkage.

Through rational structural design, the biphenyl derivatives of the present application incorporate a five-membered ring containing one or two nitrogen atoms fused at positions 2 and 3 of one benzene ring, and a substituent at position 3' of the other benzene ring that can act as a hydrogen bond donor or acceptor. These derivatives can bind well with protein targets of kinases such as PDGFRα (belonging to the TK kinase family, more specifically the RTK kinase family), TNIK (belonging to the STE kinase family), CLK4 (belonging to the CMGC kinase family, more specifically the CLK kinase family), and JNK1 (belonging to the CMGC kinase family, more specifically the MAPK kinase family), thereby simultaneously inhibiting the activity of these kinases and regulating the Wnt pathway upstream. Furthermore, they can significantly increase the expression of core reprogramming genes such as Oct4, Lin28A, and c-Myc, effectively promoting cell reprogramming. This demonstrates that simultaneous inhibition of multiple kinases upstream of the Wnt pathway can produce reprogramming effects.

In some embodiments, R¹ is selected from one of the following structures:

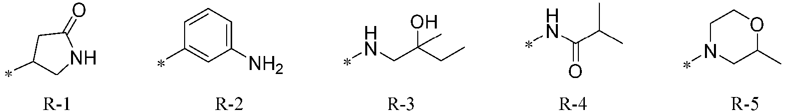

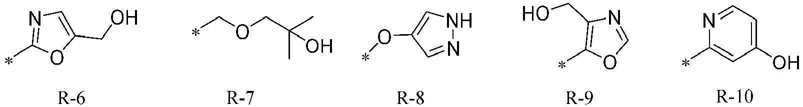

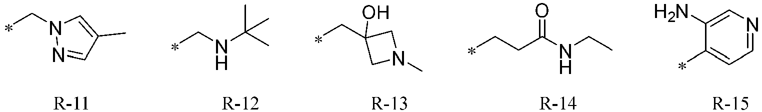

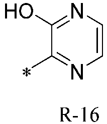

Wherein, "*" represents a point of attachment.

In some embodiments, R¹ is selected from one of the following structures:

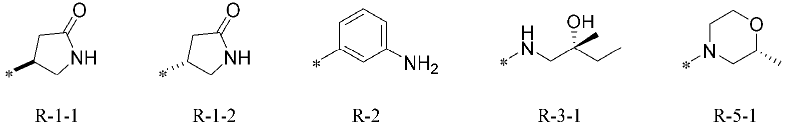

Wherein, "*" represents a point of attachment.

Preferably, R¹ is selected from R-1-1, R-1-2, R-2, or R-3-1.

In some embodiments, the A ring is selected from one of the following structures:

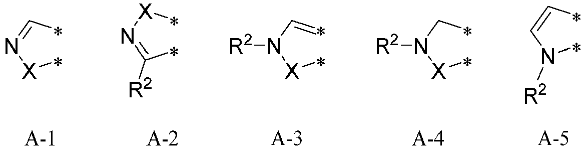

Wherein, X is CR³R⁴ or NR⁵, and R²-R⁵ are independently selected from -H, -CH₃, or -NH₂;
"*" represents a point of attachment.

In some embodiments, the A ring is selected from one of the following structures:

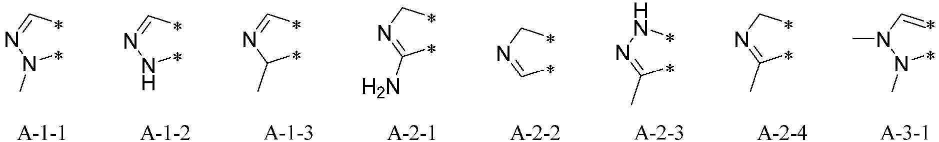

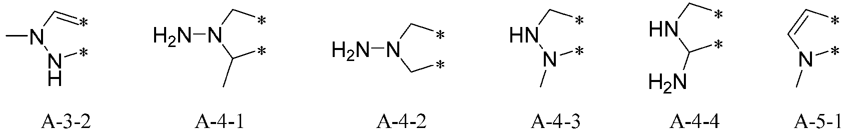

Wherein, "*" represents a point of attachment.

Preferably, the A ring is selected from one of the following structures:

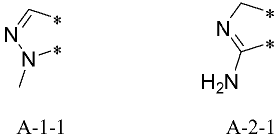

Wherein, "*" represents a point of attachment.

In some embodiments, the biphenyl derivative has a structure represented by any one of Formulas I-1 to I-18, or a pharmaceutically acceptable salt, ester, amide, solvate, active metabolite, polymorph, isotopic label, isomer, or prodrug thereof;

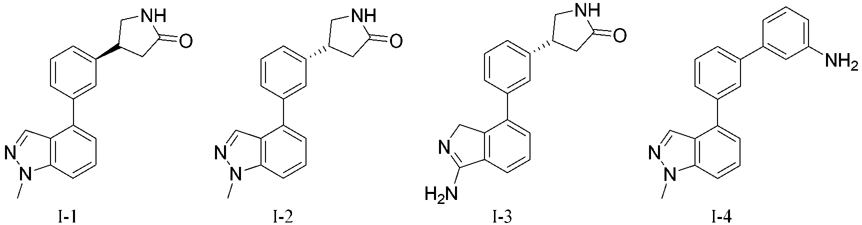

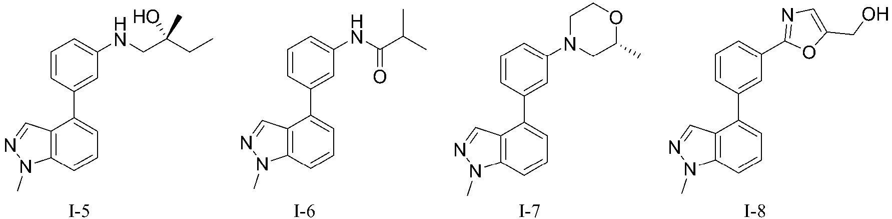

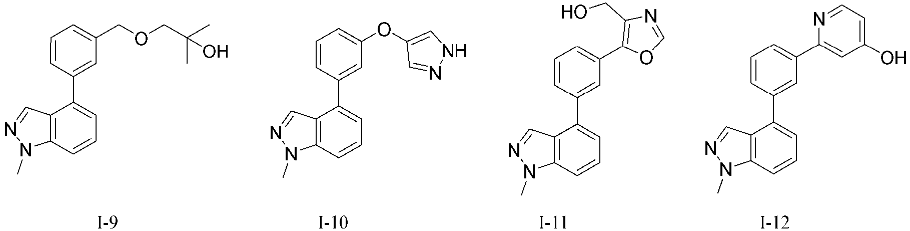

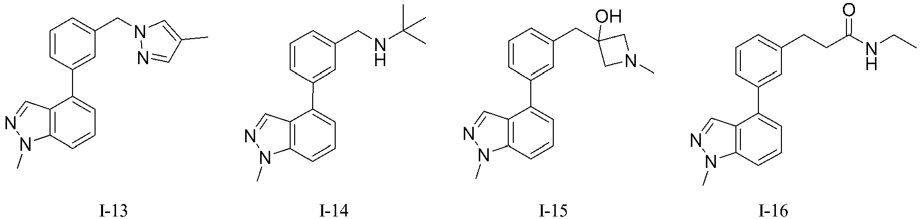

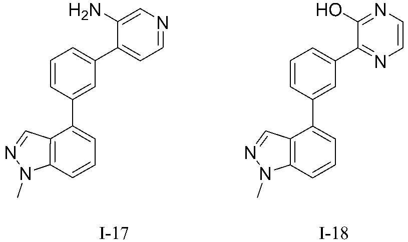

Among the more than 1900 compounds designed by the inventor of this application, 18 compounds represented by Formulas I-1 to I-18 were selected based on a comprehensive consideration of target protein calculation results, kinase inhibitory effects, and synthesis difficulty. Preferably, the compounds represented by Formulas I-1 to I-4 and I-12 exhibit better effects and are easier to synthesize.

In a second aspect, the present application provides a pharmaceutical composition comprising the biphenyl derivative of one or more of the aforementioned embodiments, and at least one pharmaceutically acceptable carrier.

In a third aspect, the present application provides the use of the biphenyl derivative of one or more of the aforementioned embodiments or the aforementioned pharmaceutical composition in cell reprogramming.

In some embodiments, when the biphenyl derivative is used for cell reprogramming, the concentration of the biphenyl derivative is 1 µM to 50 µM. Alternatively, the concentration of the biphenyl derivative may be, for example, 2 µM, 4 µM, 6 µM, 8 µM, 10 µM, 12 µM, 14 µM, 16 µM, 18 µM, 20 µM, 22 µM, 24 µM, 26 µM, 28 µM, 30 µM, 32 µM, 34 µM, 36 µM, 38 µM, 40 µM, 42 µM, 44 µM, 46 µM, or 48 µM.

In a fourth aspect, the present application provides a method for cell reprogramming, comprising the following steps:
Contacting the cells with the biphenyl derivative of one or more of the aforementioned embodiments or the aforementioned pharmaceutical composition.

In some embodiments, the concentration of the biphenyl derivative during the contacting step is from 1 µM to 50 µM. Alternatively, the concentration of the biphenyl derivative during the contacting step may be, for example, 2 µM, 4 µM, 6 µM, 8 µM, 10 µM, 12 µM, 14 µM, 16 µM, 18 µM, 20 µM, 22 µM, 24 µM, 26 µM, 28 µM, 30 µM, 32 µM, 34 µM, 36 µM, 38 µM, 40 µM, 42 µM, 44 µM, 46 µM, or 48 µM.

In a fifth aspect, the present application provides the use of the aforementioned biphenyl derivative or pharmaceutical composition in the inhibition of kinases, wherein the kinases comprise one or more of the TK kinase family, the STE kinase family, and the CMGC kinase family.

In some embodiments, the kinase comprises one or more of PDGFRα, TNIK, CLK4, and JNK1.

In a sixth aspect, the present application provides a method for inhibiting kinases, comprising the following steps:
Contacting the kinase with the biphenyl derivative of one or more of the aforementioned embodiments or the aforementioned pharmaceutical composition;
Wherein the kinase comprises one or more of the TK kinase family, STE kinase family, and CMGC kinase family.

In some embodiments, the kinase comprises one or more of PDGFRα, TNIK, CLK4, and JNK1.

### Synthesis route of compounds I-1 to I-18:

### (1) Synthesis of I-1

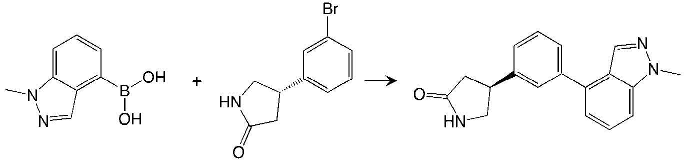

### (S)-4-(3-(1-methyl-1H-indazol-4-yl)phenyl)pyrrolidin-2-one

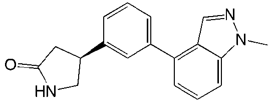

To a solution of Pd(PPh₃)₄ (1.05 g, 0.91 mmol) in ethylene glycol dimethyl ether, a solution of (S)-4-(3-bromophenyl)pyrrolidin-2-one (4.51 g, 18.77 mmol) in ethylene glycol dimethyl ether (60 mL) was added, and the mixture was stirred under nitrogen protection for 15 minutes. To the mixture from the previous step, a solution of 1-methyl-1H-indazole-4-boronic acid (5.00 g, 28.40 mmol) in ethanol (15 mL) was added, and the mixture was stirred for 10 minutes. To the resulting mixture, a 2 M Na₂CO₃ solution (80 mL) was added, and the mixture was refluxed and dried for 20 hours. After cooling the mixture, the organic layer was separated, washed with brine, dried over sodium sulfate, and concentrated under reduced pressure. The residue was purified by silica gel column chromatography (hexane: benzene = 5:1) to obtain (5)-4-(3-(1-methyl-1H-indazol-4-yl) phenyl) pyrrolidin-2-one (5.25 g, 96%), with liquid chromatography mass spectrometry m/z = 292.1 [M+H]⁺.

### (2) Synthesis of I-2

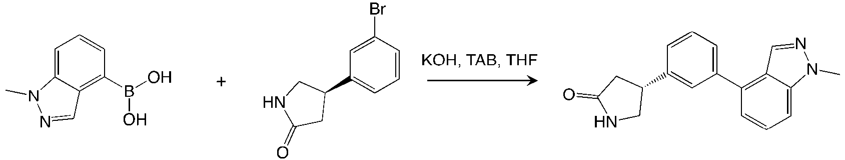

### (R)-4-(3-(1-methyl-1H-indazol-4-yl) phenyl) pyrrolidin-2-one

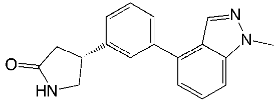

To a solution of Pd(PPh₃)₄ (1.05 g, 0.91 mmol) in ethylene glycol dimethyl ether, a solution of (R)-4-(3-bromophenyl)pyrrolidin-2-one (4.51 g, 18.77 mmol) in ethylene glycol dimethyl ether (60 mL) was added, and the mixture was stirred under nitrogen protection for 15 minutes. To the mixture from the previous step, a solution of 1-methyl-1H-indazole-4-boronic acid (5.00 g, 28.40 mmol) in ethanol (15 mL) was added, and the mixture was stirred for 10 minutes. To the resulting mixture, a 2 M Na₂CO₃ solution (80 mL) was added, and the mixture was refluxed and dried for 20 hours. After cooling the mixture, the organic layer was separated, washed with brine, dried over sodium sulfate, and concentrated under reduced pressure. The residue was purified by silica gel chromatography (hexane: benzene = 5:1) to obtain (*R*)-4-(3-(1-methyl-1H-indazol-4-yl)phenyl)pyrrolidin-2-one (5.25 g, 96%), with liquid chromatography mass spectrometry m/z = 292.1 [M+H]⁺.

### (3) Synthesis of I-3

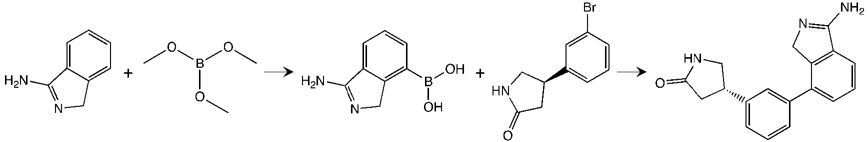

### (3-Amino-1H-isoindol-7-yl) boronic acid

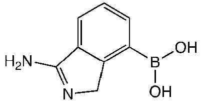

Under nitrogen protection, a solution of 1.2 mL of an alkyl lithium-tetramethylethylenediamine 1:1 complex (2.8 M hexane solution, 3.36 mmol) in anhydrous tetrahydrofuran was added to a solution of 20 mL of 1H-isoindole-3-amine (445.5 mg, 3.37 mmol) in anhydrous tetrahydrofuran within 2.5 minutes. The mixture was stirred at -78 °C for 30 minutes. To the mixture, 403.1 mg of trimethyl borate (3.88 mmol) was added, and the mixture was stirred at -78 °C for 5 minutes before the cryostat reaction bath was stopped. After the mixture had returned to room temperature, the tetrahydrofuran was removed under vacuum. A 5% aqueous HCl solution was added, and the mixture was washed with diethyl ether. The organic layer was separated, dried over magnesium sulfate, and concentrated under reduced pressure. The residue was purified by silica gel chromatography (ethyl acetate: hexane = 3:7) to obtain (3-amino-1H-isoindol-7-yl)boronic acid (474.5 mg, 80%), with liquid chromatography-mass spectrometry m/z = 177.1 [M+H]⁺.

### (S)-4-(3-(3-amino-1H-isoindol-7-yl)phenyl)pyrrolidin-2-one

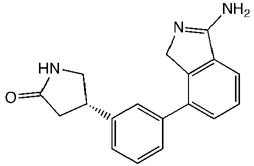

To a solution of Pd(PPh₃)₄ (1.05 g, 0.91 mmol) in ethylene glycol dimethyl ether, a solution of (R)-4-(3-bromophenyl)pyrrolidin-2-one (4.51 g, 18.77 mmol) in ethylene glycol dimethyl ether (60 mL) was added, and the mixture was stirred under nitrogen protection for 15 minutes. To the mixture from the previous step, a solution of (3-amino-1H-isoindol-7-yl)boronic acid (5.00 g, 28.40 mmol) in ethanol (15 mL) was added, and the mixture was stirred for 10 minutes. To the resulting mixture, a 2 M Na₂CO₃ solution (80 mL) was added, and the mixture was refluxed and dried for 20 hours. After cooling the mixture, the organic layer was separated, washed with brine, dried over sodium sulfate, and concentrated under reduced pressure. The residue was purified by silica gel chromatography (hexane: benzene = 5:1) to obtain (*R*)-4-(3-(3-amino-1H-isoindol-7-yl) phenyl) pyrrolidin-2-one (5.25 g, 96%), with liquid chromatography mass spectrometry m/z = 292.1 [M+H]⁺.

### (4) Synthesis of I-4

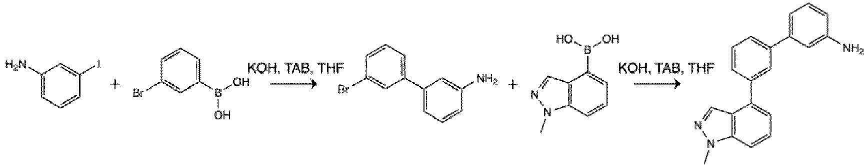

### 3'-Bromo-[1,1'-biphenyl]-3-amine

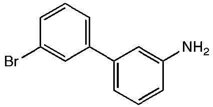

To a solution of Pd(PPh₃)₄ (1.05 g, 0.91 mmol) in ethylene glycol dimethyl ether, a solution of 3-iodoaniline (4.10 g, 18.77 mmol) in ethylene glycol dimethyl ether (60 mL) was added, and the mixture was stirred under nitrogen protection for 15 minutes. To the mixture from the previous step, a solution of 3-bromophenylboronic acid (5.70 g, 28.40 mmol) in ethanol (15 mL) was added, and the mixture was stirred for 10 minutes. To the resulting mixture, a 2 M Na₂CO₃ solution (80 mL) was added, and the mixture was refluxed and dried for 20 hours. After cooling the mixture, the organic layer was separated, washed with brine, dried over sodium sulfate, and concentrated under reduced pressure. The residue was purified by silica gel chromatography (hexane: benzene = 5:1) to obtain 3'-bromo-[1,1'-biphenyl]-3-amine (3.12 g, 67%), with liquid chromatography mass spectrometry m/z = 248.0 [M+H]⁺.

### 3'-(1-methyl-1H-indazol-4-yl)-[1,1'-biphenyl]-3-amine

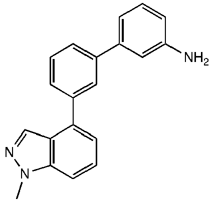

To a solution of Pd(PPh₃)₄ (1.05 g, 0.91 mmol) in ethylene glycol dimethyl ether, a solution of 3'-bromo-[1,1'-biphenyl]-3-amine (4.66 g, 18.77 mmol) in ethylene glycol dimethyl ether (60 mL) was added, and the mixture was stirred under nitrogen protection for 15 minutes. To the mixture from the previous step, a solution of (1-methyl-1H-indazol-4-yl)boronic acid (5.00 g, 28.40 mmol) in ethanol (15 mL) was added, and the mixture was stirred for 10 minutes. To the resulting mixture, a 2 M Na₂CO₃ solution (80 mL) was added, and the mixture was refluxed and dried for 20 hours. After cooling the mixture, the organic layer was separated, washed with brine, dried over sodium sulfate, and concentrated under reduced pressure. The residue was purified by silica gel chromatography (hexane: benzene = 5:1) to obtain 3'-(1-methyl-1H-indazol-4-yl)-[1,1'-biphenyl]-3-amine (5.39 g, 96%), with liquid chromatography-mass spectrometry m/z = 300.1 [M+H]⁺.

### (5) Synthesis of I-5

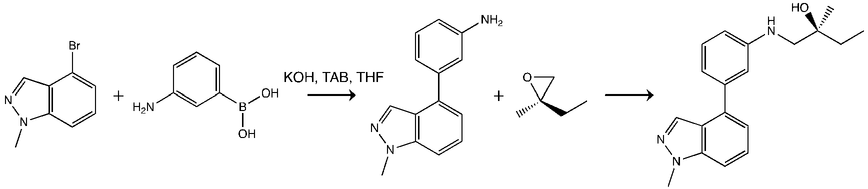

### 3-(1-Methyl-1H-indazol-4-yl)aniline

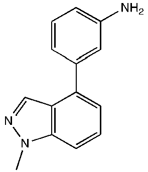

To a solution of Pd(PPh₃)₄ (1.05 g, 0.91 mmol) in ethylene glycol dimethyl ether, a solution of 4-bromo-1-methyl-1H-indazole (3.96 g, 18.77 mmol) in ethylene glycol dimethyl ether (60 mL) was added, and the mixture was stirred under nitrogen protection for 15 minutes. To the mixture from the previous step, a solution of 3-aminobenzeneboronic acid (3.89 g, 28.40 mmol) in ethanol (15 mL) was added, and the mixture was stirred for 10 minutes. To the resulting mixture, a 2 M Na₂CO₃ solution (80 mL) was added, and the mixture was refluxed and dried for 20 hours. After cooling the mixture, the organic layer was separated, washed with brine, dried over sodium sulfate, and concentrated under reduced pressure. The residue was purified by silica gel chromatography (hexane: benzene = 5:1) to obtain 3-(1-methyl-1H-indazol-4-yl)aniline (4.02 g, 96%), with liquid chromatography mass spectrometry m/z = 224.1 [M+H]⁺.

### (S)-2-methyl-1-((3-(1-methyl-1H-indazol-4-yl)phenyl)amino)-2-butanol

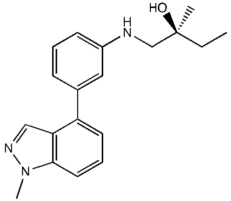

To a 20 mL pressure tube, add (R)-2-ethyl-2-methyl oxirane (86.1 mg, 1.0 mmol), 3-(1-methyl-1H-indazol-4-yl)aniline (335.0 mg, 1.5 mmol), and 6.7 mL of reagent-grade DMF. Seal the mixture and stir at 60°C for 12 hours. After cooling the reaction to room temperature, add 6.7 mL of deionized water, and seal the mixture and stir at 60°C for another 12 hours. Remove the solvent using a rotary evaporator (22.5 mbar, 35°C), and purify the residue via silica gel chromatography (ethyl acetate: hexane = 1:2) to obtain (S)-2-methyl-1-((3-(1-methyl-1H-indazol-4-yl)phenyl)amino)-2-butanol (281.6 mg, 91%), with liquid chromatography mass spectrometry m/z = 310.2 [M+H]⁺.

### (6) Synthesis of I-6

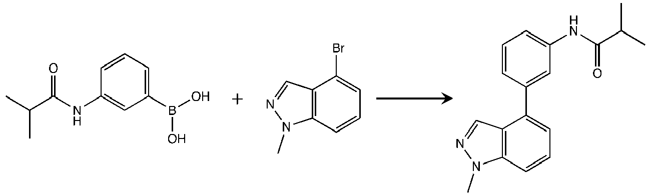

### N-(3-(1-methyl-1H-indazol-4-yl)phenyl)isobutyramide

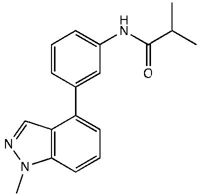

To a solution of Pd(PPh₃)₄ (1.05 g, 0.91 mmol) in ethylene glycol dimethyl ether, a solution of 4-bromo-1-methyl-1H-indazole (3.96 g, 18.77 mmol) in ethylene glycol dimethyl ether (60 mL) was added, and the mixture was stirred under nitrogen protection for 15 minutes. To the mixture from the previous step, a solution of 3-isobutyrylamidophenylboronic acid (5.88 g, 28.40 mmol) in ethanol (15 mL) was added, and the mixture was stirred for 10 minutes. To the resulting mixture, a 2 M Na₂CO₃ solution (80 mL) was added, and the mixture was refluxed and dried for 20 hours. After cooling the mixture, the organic layer was separated, washed with brine, dried over sodium sulfate, and concentrated under reduced pressure. The residue was purified by silica gel chromatography (hexane: benzene = 5:1) to obtain N-(3-(1-methyl-1H-indazol-4-yl)phenyl)isobutyramide (5.29 g, 96%), with liquid chromatography mass spectrometry m/z = 294.2 [M+H]⁺.

### (7) Synthesis of I-7

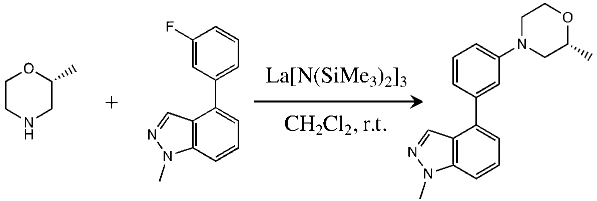

### (R)-2-methyl-4-(3-(1-methyl-1H-indazol-4-yl)phenyl)morpholine

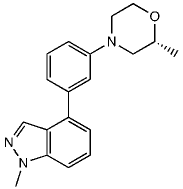

To La[N(SiMe₃)₂]₃ (124 mg, 0.20 mmol, stored and weighed in a fume hood), CH₂Cl₂ (1.25 mL), 4-(3-fluorophenyl)-1-methyl-1H-indazole (200 µL, 0.20 mmol, 1.0 M solution in CH₂Cl₂), and (R)-2-methylmorpholine (37 µL, 0.22 mmol) were added. The mixed system was stirred at room temperature for 1 minute, and the residue was purified by silica gel chromatography (CH₂Cl₂ MeOH = 95:5) to obtain (R)-2-methyl-4-(3-(1-methyl-1H-indazol-4-yl)phenyl)morpholine (57.2 mg, 93%), with liquid chromatography mass spectrometry m/z = 308.2 [M+H]⁺.

### (8) Synthesis of I-8

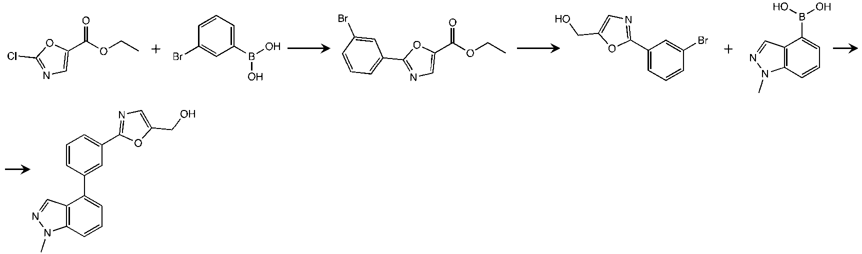

### Ethyl 2-(3-bromophenyl)oxazole-5-carboxylate

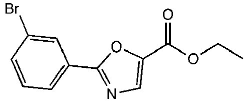

To a solution of Pd(PPh₃)₄ (1.05 g, 0.91 mmol) in ethylene glycol dimethyl ether, a solution of 2-chlorooxazole-5-carboxylic acid ethyl ester (3.30 g, 18.77 mmol) in ethylene glycol dimethyl ether (60 mL) was added, and the mixture was stirred under nitrogen protection for 15 minutes. To the mixture from the previous step, a solution of 3-bromophenylboronic acid (5.70 g, 28.40 mmol) in ethanol (15 mL) was added, and the mixture was stirred for 10 minutes. To the resulting mixture, a 2 M Na₂CO₃ solution (80 mL) was added, and the mixture was refluxed and dried for 20 hours. After cooling the mixture, the organic layer was separated, washed with brine, dried over sodium sulfate, and concentrated under reduced pressure. The residue was purified by silica gel chromatography (hexane: benzene = 5:1) to obtain Ethyl 2-(3-bromophenyl)oxazole-5-carboxylate (2.72 g, 49%), with liquid chromatography mass spectrometry m/z = 296.0 [M+H]⁺.

### (2-(3-bromophenyl)oxazol-5-yl)methanol

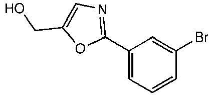

To a methanol solution (100 mL, 0°C) of 2-(3-bromophenyl)oxazole-5-carboxylic acid ethyl ester (11.4 g, 38.6 mmol), NaBH₄ (4.4 g, 115.8 mmol, 3 eq) was added in batches. The mixture was stirred at room temperature for 3 hours, and then water (100 mL) was added to terminate the reaction. The product was extracted using ethyl acetate (3 × 40 mL), and the organic layer was separated, washed with brine, dried over sodium sulfate, and concentrated under reduced pressure to obtain (2-(3-bromophenyl)oxazol-5-yl)methanol (9.3 g, 95%), with liquid chromatography mass spectrometry m/z = 254.0 [M+H]⁺.

### (2-(3-(1-methyl-1H-indazol-4-yl)phenyl)oxazol-5-yl)methanol

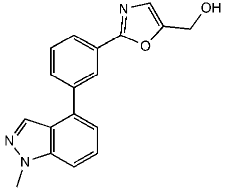

To a solution of Pd(PPh₃)₄ (1.05 g, 0.91 mmol) in ethylene glycol dimethyl ether, a solution of (2-(3-bromophenyl)oxazol-5-yl)methanol (4.77 g, 18.77 mmol) in ethylene glycol dimethyl ether (60 mL) was added, and the mixture was stirred under nitrogen protection for 15 minutes. To the mixture from the previous step, a solution of (1-methyl-1H-indazol-4-yl)boronic acid (5.00 g, 28.40 mmol) in ethanol (15 mL) was added, and the mixture was stirred for 10 minutes. To the resulting mixture, a 2 M Na₂CO₃ solution (80 mL) was added, and the mixture was refluxed and dried for 20 hours. After cooling the mixture, the organic layer was separated, washed with brine, dried over sodium sulfate, and concentrated under reduced pressure. The residue was purified by silica gel column chromatography (hexane: benzene = 5:1) to obtain (2-(3-(1-methyl-1H-indazol-4-yl)phenyl)oxazol-5-yl)methanol (5.50 g, 96%), with liquid chromatography mass spectrometry m/z = 306.1 [M+H]⁺.

### (9) Synthesis of I-9

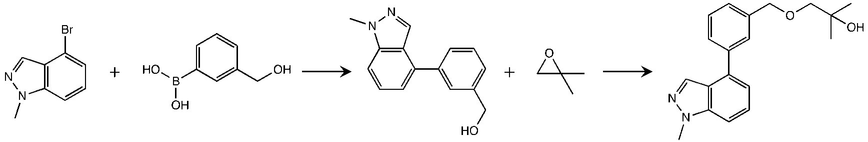

### (3-(1-methyl-1H-indazol-4-yl)phenyl)methanol

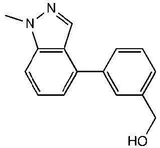

To a solution of Pd(PPh₃)₄ (1.05 g, 0.91 mmol) in ethylene glycol dimethyl ether, a solution of 4-bromo-1-methyl-1H-indazole (3.96 g, 18.77 mmol) in ethylene glycol dimethyl ether (60 mL) was added, and the mixture was stirred under nitrogen protection for 15 minutes. To the mixture from the previous step, a solution of 3-hydroxymethylphenylboronic acid (4.32 g, 28.40 mmol) in ethanol (15 mL) was added, and the mixture was stirred for 10 minutes. To the resulting mixture, a 2 M Na₂CO₃ solution (80 mL) was added, and the mixture was refluxed and dried for 20 hours. After cooling the mixture, the organic layer was separated, washed with brine, dried over sodium sulfate, and concentrated under reduced pressure. The residue was purified by silica gel column chromatography (hexane: benzene = 5:1) to obtain (3-(1-methyl-1H-indazol-4-yl)phenyl)methanol (4.29 g, 96%), with liquid chromatography mass spectrometry (LCMS) m/z = 239.1 [M+H]⁺.

### 2-Methyl-1-((3-(1-methyl-1H-indazol-4-yl)phenyl)oxy)isopropanol

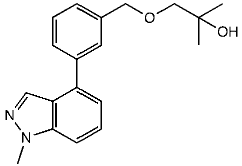

To a solution of 2,2-epoxybutane (15.0 mL, 168 mmol) and (3-(1-methyl-1H-indazol-4-yl)phenyl)methanol (11.0 g, 46 mmol) in toluene (40 mL), 50% aq. NaOH (12 mL) was added, and the mixture was stirred at 100°C for 30 hours. Water and ethyl acetate were added to the mixture, and the organic layer was separated, washed with brine, dried over sodium sulfate, and concentrated under reduced pressure. The residue was purified by silica gel chromatography (petroleum ether: ethyl acetate = 5:1) to obtain 2-methyl-1-((3-(1-methyl-1H-indazol-4-yl)phenyl)oxy)isopropanol (14.2 g, 99%), LCMS m/z = 311.2 [M+H]⁺.

### (10) Synthesis of I-10

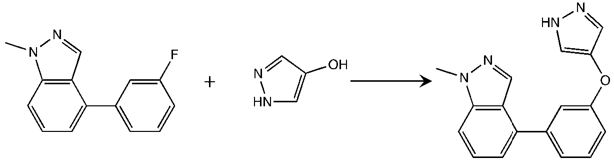

### 4-(3-((1H-pyrazol-4-yl)oxy)phenyl)-1-methyl-1H-indazole

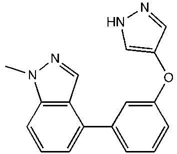

To 4-hydroxypyrazole (849.4 mg, 10.1 mmol), sodium hydride (60%, 256.8 mg, 10.7 mmol) was added. After 15 minutes, 4-(3-fluorophenyl)-1-methyl-1H-indazole (2.3 g, 10.2 mmol) was added, and the mixture was stirred at room temperature for 2 hours. The product was extracted with ethyl acetate, and the organic layer was separated and dried. The residue was purified by silica gel chromatography (ethyl acetate: petroleum ether = 1:1) to obtain 4-(3-((1H-pyrazol-4-yl)oxy)phenyl)-1-methyl-1H-indazole (1.47 g, 50%), with liquid chromatography mass spectrometry m/z = 291.1 [M+H]⁺.

### (11) Synthesis of I-11

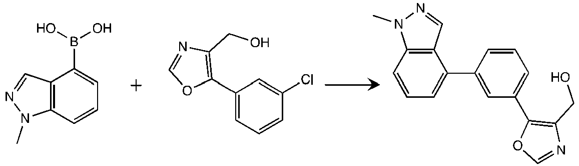

### (5-(3-(1-methyl-1H-indazol-4-yl)phenyl)oxazol-4-yl)methanol

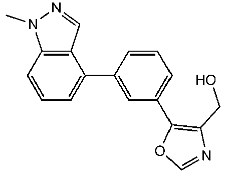

To a solution of Pd(PPh₃)₄ (1.05 g, 0.91 mmol) in ethylene glycol dimethyl ether, a solution of (5-(3-chlorophenyl)oxazol-4-yl)methanol (3.93 g, 18.77 mmol) in ethylene glycol dimethyl ether (60 mL) was added, and the mixture was stirred under nitrogen protection for 15 minutes. To the mixture from the previous step, a solution of (1-methyl-1H-indazol-4-yl)boronic acid (5.00 g, 28.40 mmol) in ethanol (15 mL) was added, and the mixture was stirred for 10 minutes. To the resulting mixture, a 2 M Na₂CO₃ solution (80 mL) was added, and the mixture was refluxed and dried for 20 hours. After cooling, the organic layer was separated, washed with brine, dried over sodium sulfate, and concentrated under reduced pressure. The residue was purified by silica gel chromatography (hexane: benzene = 5:1) to obtain (5-(3-(1-methyl-1H-indazol-4-yl)phenyl)oxazol-4-yl)methanol (5.50 g, 96%), with liquid chromatography mass spectrometry m/z = 306.1 [M+H]⁺.

### (12) Synthesis of I-12

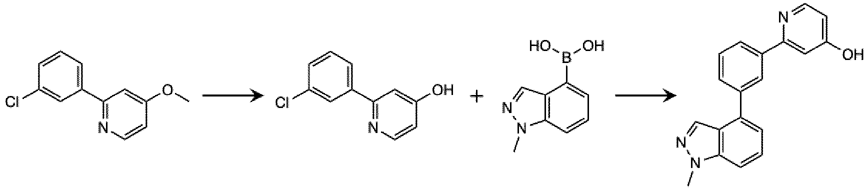

### 2-(3-chlorophenyl)-4-hydroxypyridine

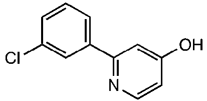

Under stirring conditions, Aliquat-336 (10% of the substrate weight) was added in one portion to a mixture of 2-(3-chlorophenyl)-4-methoxypyridine (20 mmol) and 47% HBr (4.5 mmol). The mixture was heated to 105 ± 5 °C and the reaction progress was continuously monitored through TLC (thin-layer chromatography) experiments. After the reaction was complete, the mixture was cooled to room temperature and water (25 mL) was added to terminate the reaction. The product was extracted using ethyl acetate (3 × 30 mL), the organic layer was separated, washed with brine, dried over sodium sulfate, and concentrated under reduced pressure. The residue was purified by silica gel chromatography (hexane: ethyl acetate = 2:1) to obtain 2-(3-chlorophenyl)-4-hydroxypyridine (3.94 g, 96%), with liquid chromatography mass spectrometry m/z = 206.0 [M+H]⁺.

### 2-(3-(1-Methyl-1H-indazol-4-yl)phenyl)-4-hydroxypyridine

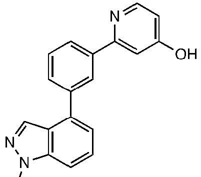

To a solution of Pd(PPh₃)₄ (1.05 g, 0.91 mmol) in ethylene glycol dimethyl ether, a solution of 2-(3-chlorophenyl)-4-hydroxypyridine (3.86 g, 18.77 mmol) in ethylene glycol dimethyl ether (60 mL) was added, and the mixture was stirred under nitrogen protection for 15 minutes. To the mixture from the previous step, a solution of (1-methyl-1H-indazol-4-yl)boronic acid (5.00 g, 28.40 mmol) in ethanol (15 mL) was added, and the mixture was stirred for 10 minutes. To the resulting mixture, a 2 M Na₂CO₃ solution (80 mL) was added, and the mixture was refluxed and dried for 20 hours. After cooling the mixture, the organic layer was separated, washed with brine, dried over sodium sulfate, and concentrated under reduced pressure. The residue was purified by silica gel chromatography (hexane: benzene = 5:1) to obtain 2-(3-(1-methyl-1H-indazol-4-yl)phenyl)-4-hydroxypyridine (5.43 g, 96%), with liquid chromatography mass spectrometry m/z = 302.1 [M+H]⁺.

### (13) Synthesis of I-13

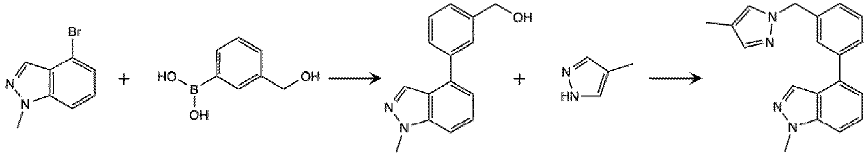

### (3-(1-methyl-1H-indazol-4-yl)phenyl)methanol

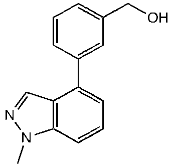

To a solution of Pd(PPh₃)₄ (1.05 g, 0.91 mmol) in ethylene glycol dimethyl ether, a solution of 4-bromo-1-methyl-1H-indazole (3.96 g, 18.77 mmol) in ethylene glycol dimethyl ether (60 mL) was added, and the mixture was stirred under nitrogen protection for 15 minutes. To the mixture from the previous step, a solution of 3-hydroxymethylphenylboronic acid (4.32 g, 28.40 mmol) in ethanol (15 mL) was added, and the mixture was stirred for 10 minutes. To the resulting mixture, a 2 M Na₂CO₃ solution (80 mL) was added, and the mixture was refluxed and dried for 20 hours. After cooling the mixture, the organic layer was separated, washed with brine, dried over sodium sulfate, and concentrated under reduced pressure. The residue was purified by silica gel chromatography (hexane: benzene = 5:1) to obtain (3-(1-methyl-1H-indazol-4-yl)phenyl)methanol (4.29 g, 96%), with liquid chromatography-mass spectrometry m/z = 239.1 [M+H]⁺.

### 1-Methyl-4-(3-((4-methyl-1H-pyrazol-1-yl)methyl)phenyl)-1H-indazole

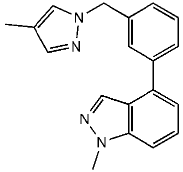

To a 100 mL round-bottom flask, add 2.38 g (10 mmol) of (3-(1-methyl-1H-indazol-4-yl)phenyl)methanol, 1.64 g (20 mmol) of 4-methyl-1H-pyrazole, and 0.26 g (1 mmol) of Ni(ClO₄)₂. Finally, add 20 mL of anhydrous 1,2-dichloroethane, and stir the mixture at 85 °C for 6 hours. After cooling the mixture to room temperature, pour it into an ice-water mixture. Extract the product with dichloromethane (3 × 50 mL), separate the organic layer, wash with brine, dry over sodium sulfate, and concentrate under reduced pressure. The residue is purified by silica gel chromatography (hexane: benzene = 5:1) to obtain 1-methyl-4-(3-((4-methyl-1H-pyrazol-1-yl)methyl)phenyl)-1H-indazole (2.78 g, 92%), with liquid chromatography mass spectrometry m/z = 303.1 [M+H]⁺.

### (14) Synthesis of I-14

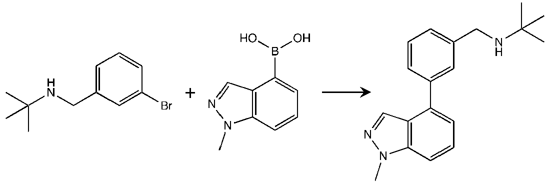

### 2-Methyl-N-(3-(1-methyl-1H-indazol-4-yl)phenyl)isopropylamine

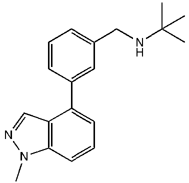

To a solution of Pd(PPh₃)₄ (1.05 g, 0.91 mmol) in ethylene glycol dimethyl ether, a solution of N-(3-bromobenzyl)-2-methylisopropylamine (4.55 g, 18.77 mmol) in ethylene glycol dimethyl ether (60 mL) was added, and the mixture was stirred under nitrogen protection for 15 minutes. To the mixture from the previous step, a solution of (1-methyl-1H-indazol-4-yl)boronic acid (5.00 g, 28.40 mmol) in ethanol (15 mL) was added, and the mixture was stirred for 10 minutes. To the resulting mixture, a 2 M Na₂CO₃ solution (80 mL) was added, and the mixture was refluxed and dried for 20 hours. After cooling the mixture, the organic layer was separated, washed with brine, dried over sodium sulfate, and concentrated under reduced pressure. The residue was purified by silica gel chromatography (hexane: benzene = 5:1) to obtain 2-methyl-N-(3-(1-methyl-1H-indazol-4-yl)phenyl)isopropylamine (5.29 g, 96%), with liquid chromatography mass spectrometry m/z = 294.2 [M+H]⁺.

### (15) Synthesis of I-15

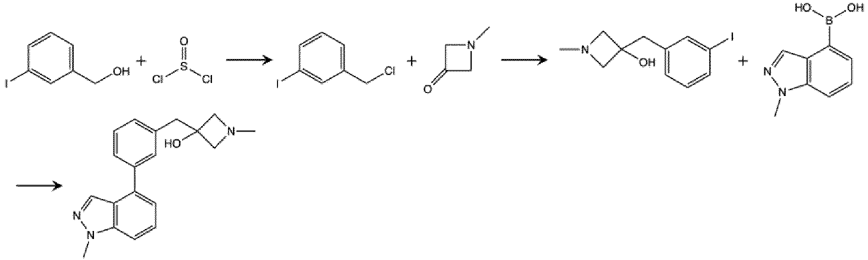

### 1-(chloromethyl)-3-iodobenzene

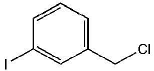

Under nitrogen protection, (3-iodophenyl)methanol (41.2 g), toluene (300 mL), and pyridine (0.5 mL) were mixed and stirred at 45°C for 1 hour. Within the temperature range of 45-55°C, thionyl chloride (14.0 mL) was added to the mixture, and the mixture was heated under reflux for 2 hours. The mixture was cooled to 25°C, water (300 mL) and toluene (300 mL) were added and mixed, the organic layer was separated, washed with brine, dried over sodium sulfate, and concentrated under reduced pressure. The residue was purified by silica gel column chromatography (n-heptane: toluene = 1:1) to obtain 1-(chloromethyl)-3-iodobenzene (42.79 g, 94%), with liquid chromatography mass spectrometry m/z = 252.9 [M+H]⁺.

### 3-(3-iodophenyl)-1-methylazetidin-3-ol

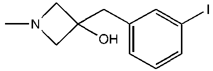

To a solution of magnesium powder (3.2 g, 131.66 mmol) in diethyl ether (190 mL), a solution of 1-(chloromethyl)-3-iodobenzene (31.97 g, 126.62 mmol) in diethyl ether (32 mL) was added, and the mixture was stirred at 45°C for 4 hours. After cooling the reaction mixture to room temperature, a solution of 1-methylazetidin-3-one (16.81 g, 197.49 mmol) in diethyl ether (32 mL) at 0°C was added, and the mixture was stirred at room temperature overnight. The mixture was placed in ice, and dilute hydrochloric acid (60 mL) and NaHSO₄ solution (1.0 M, 50 mL) were added. The product was extracted with ethyl acetate (3 × 100 mL), the organic layer was separated, washed with brine, dried over sodium sulfate, and concentrated under reduced pressure. The residue was purified by silica gel chromatography (ethyl acetate: petroleum ether = 1:1) to obtain 3-(3-iodophenyl)-1-methylazetidin-3-ol (36.46 g, 95%), with liquid chromatography mass spectrometry m/z = 304.0 [M+H]⁺.

### 1-Methyl-3-(3-(1-methyl-1H-indazol-4-yl)phenyl)azetidin-3-ol

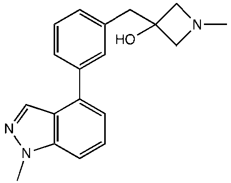

To a solution of Pd(PPh₃)₄ (1.05 g, 0.91 mmol) in ethylene glycol dimethyl ether, a solution of 3-(3-iodophenyl)-1-methylazetidin-3-ol (5.69 g, 18.77 mmol) in ethylene glycol dimethyl ether (60 mL) was added, and the mixture was stirred under nitrogen protection for 15 minutes. To the mixture from the previous step, a solution of (1-methyl-1H-indazol-4-yl)boronic acid (5.00 g, 28.40 mmol) in ethanol (15 mL) was added, and the mixture was stirred for 10 minutes. To the resulting mixture, a 2 M Na₂CO₃ solution (80 mL) was added, and the mixture was refluxed and dried for 20 hours. After cooling the mixture, the organic layer was separated, washed with brine, dried over sodium sulfate, and concentrated under reduced pressure. The residue was purified by silica gel column chromatography (hexane: benzene = 5:1) to obtain 1-methyl-3-(3-(1-methyl-1H-indazol-4-yl)phenyl)azetidin-3-ol (5.54 g, 96%), with liquid chromatography mass spectrometry m/z = 308.2 [M+H]⁺.

### (16) Synthesis of I-16

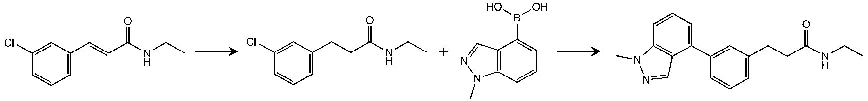

### 3-(3-chlorophenyl)-N-ethylpropionamide

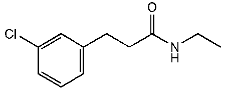

Under nitrogen protection, a diethyl ether solution of methyllithium (1.6 M, 0.8 mL, 0.5 mmol) was added to a mixture of copper(I) iodide and anhydrous tetrahydrofuran (12 mL), and the mixture was stirred in an ice bath for 15 minutes. The ice bath was replaced with -78°C, and while stirring, anhydrous hexamethylphosphoric triamide (1.7 mL, 10 mmol) was added, followed by a cyclohexane solution of diisobutyl aluminum hydride (1 M, 8.0 mL, 8 mmol). The mixture was stirred at this temperature for 70 minutes. (E)-3-(3-chlorophenyl)-N-ethylacrylamide (1.0 mmol) was added, and the mixture was stirred at this temperature for 70 minutes. Methanol (5 mL) was added, and after the mixture naturally returned to room temperature, saturated potassium sodium citrate (20 mL) and diethyl ether (30 mL) were added. The mixture was stirred at room temperature overnight. The organic layer was separated, washed with brine, dried over sodium sulfate, and concentrated under reduced pressure. The residue was purified by silica gel chromatography (ethyl acetate: petroleum ether = 1:1) to obtain 3-(3-chlorophenyl)-N-ethylacrylamide (203.2 mg, 96%), with liquid chromatography mass spectrometry m/z = 212.1 [M+H]⁺.

### N-ethyl-3-(3-(1-methyl-1H-indazol-4-yl)phenyl)propionamide

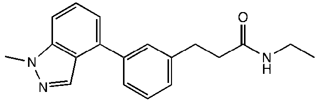

To a solution of Pd(PPh₃)₄ (1.05 g, 0.91 mmol) in ethylene glycol dimethyl ether, a solution of 3-(3-chlorophenyl)-N-ethylpropionamide (3.97 g, 18.77 mmol) in ethylene glycol dimethyl ether (60 mL) was added, and the mixture was stirred under nitrogen protection for 15 minutes. To the previous mixture, a solution of (1-methyl-1H-indazol-4-yl)boronic acid (5.00 g, 28.40 mmol) in ethanol (15 mL) was added, and the mixture was stirred for 10 minutes. To the resulting mixture, a 2 M Na₂CO₃ solution (80 mL) was added, and the mixture was refluxed and dried for 20 hours. After cooling the mixture, the organic layer was separated, washed with brine, dried over sodium sulfate, and concentrated under reduced pressure. The residue was purified by silica gel chromatography (hexane: benzene = 5:1) to obtain N-ethyl-3-(3-(1-methyl-1H-indazol-4-yl)phenyl)propionamide (5.54 g, 96%), with liquid chromatography mass spectrometry m/z = 308.2 [M+H]⁺.

### (17) Synthesis of I-17

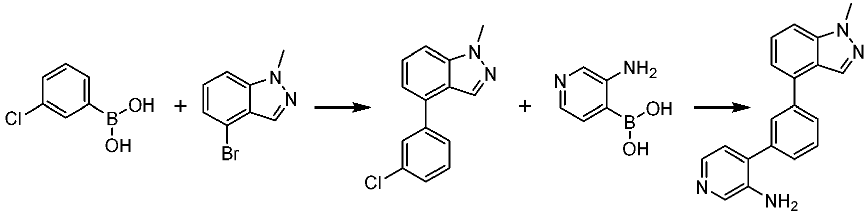

### 4-(3-chlorophenyl)-1-methylindazole

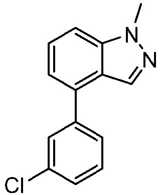

To a solution of Pd(PPh₃)₄ (1.05 g, 0.91 mmol) in ethylene glycol dimethyl ether, a solution of 4-bromo-1-methyl-1H-indazole (3.96 g, 18.77 mmol) in ethylene glycol dimethyl ether (60 mL) was added, and the mixture was stirred under nitrogen protection for 15 minutes. To the previous mixture, a solution of (3-chlorophenyl)boronic acid (4.44 g, 28.40 mmol) in ethanol (15 mL) was added, and the mixture was stirred for 10 minutes. To the resulting mixture, a 2 M Na₂CO₃ solution (80 mL) was added, and the mixture was refluxed and dried for 20 hours. After cooling the mixture, the organic layer was separated, washed with brine, dried over sodium sulfate, and concentrated under reduced pressure. The residue was purified by silica gel chromatography (hexane: benzene = 5:1) to obtain 4-(3-chlorophenyl)-1-methylindazole (4.37 g, 96%), with liquid chromatography mass spectrometry m/z = 243.1 [M+H]⁺.

### 4-(3-(1-methylindazol-4-yl)phenyl)pyridin-3-amine

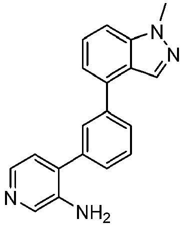

To a solution of Pd(PPh₃)₄ (1.05 g, 0.91 mmol) in ethylene glycol dimethyl ether, a solution of 4-(3-chlorophenyl)-1-methylindazole (4.37 g, 18.02 mmol) in ethylene glycol dimethyl ether (60 mL) was added, and the mixture was stirred under nitrogen protection for 15 minutes. To the mixture from the previous step, a solution of (3-aminopyridin-4-yl)boronic acid (3.92 g, 28.40 mmol) in ethanol (15 mL) was added, and the mixture was stirred for 10 minutes. To the resulting mixture, a 2 M Na₂CO₃ solution (80 mL) was added, and the mixture was refluxed and dried for 20 hours. After cooling the mixture, the organic layer was separated, washed with brine, dried over sodium sulfate, and concentrated under reduced pressure. The residue was purified by silica gel column chromatography (hexane: benzene = 5:1) to obtain 4-(3-(1-methylindol-4-yl)phenyl)pyridin-3-amine (5.19 g, 96%), with liquid chromatography mass spectrometry m/z = 301.1 [M+H]⁺.

### (18) Synthesis of I-18

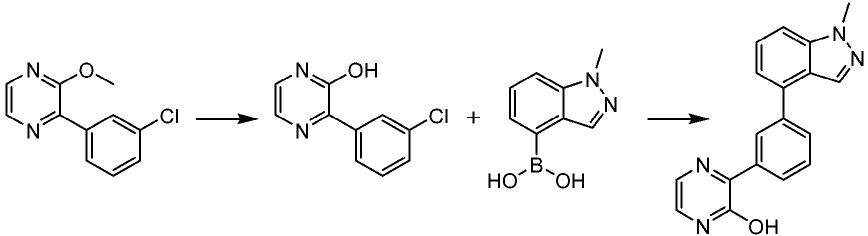

### 3-(3-chlorophenyl)pyrazin-2-ol

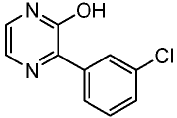

Under stirring conditions, Aliquat-336 (10% of the substrate weight) was added in one portion to a mixture of 2-(3-chlorophenyl)-3-methoxypyrazine (20 mmol) and 47% HBr (4.5 mmol). The mixture was heated to 105 ± 5 °C, and the reaction progress was continuously monitored through TLC experiments. After the reaction was complete, the mixture was cooled to room temperature, and water (25 mL) was added to terminate the reaction. The product was extracted using ethyl acetate (3 × 30 mL), and the organic layer was separated, washed with brine, dried over sodium sulfate, and concentrated under reduced pressure. The residue was purified by silica gel chromatography (hexane: ethyl acetate = 2:1) to obtain 3-(3-chlorophenyl)pyrazin-2-ol (3.96 g, 96%), with liquid chromatography mass spectrometry m/z = 207.0 [M+H]⁺.

### 3-(3-(1-methylindazol-4-yl)phenyl)pyrazin-2-ol

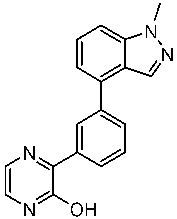

To a solution of Pd(PPh₃)₄ (1.05 g, 0.91 mmol) in ethylene glycol dimethyl ether, a solution of 3-(3-chlorophenyl)pyrazin-2-ol (3.87 g, 18.77 mmol) in ethylene glycol dimethyl ether (60 mL) was added, and the mixture was stirred under nitrogen protection for 15 minutes. To the mixture from the previous step, a solution of (1-methyl-1H-indazol-4-yl)boronic acid (5.00 g, 28.40 mmol) in ethanol (15 mL) was added, and the mixture was stirred for 10 minutes. To the resulting mixture, a 2 M Na₂CO₃ solution (80 mL) was added, and the mixture was refluxed and dried for 20 hours. After cooling the mixture, the organic layer was separated, washed with brine, dried over sodium sulfate, and concentrated under reduced pressure. The residue was purified by silica gel chromatography (hexane: benzene = 5:1) to obtain 3-(3-(1-methylindazol-4-yl)phenyl)pyrazin-2-ol (5.44 g, 96%), with liquid chromatography mass spectrometry m/z = 303.1 [M+H]⁺.

### Example 1: Virtual screening of target kinases and biphenyl derivatives

Virtual screening is a computational technique used in drug discovery to search small molecule compound libraries for chemical small molecules that may bind to drug targets through computational methods. Compared to traditional high-throughput drug screening techniques, virtual screening offers advantages such as high efficiency and cost-effectiveness. Therefore, this application employs virtual screening to predict the effects of compounds. The specific theory and methodology are based on "Docking Screens for Novel Ligands Conferring New Biology" (Irwin JJ and Shoichet BK, J Med Chem. 2016 May 12;59(9):4103-20).

Comparative analysis of protein structures reveals that PDGFR, TNIK, CLK4, and CLK3 share similar three-dimensional structures (RMSD ≤ 8.4), with the structural similarity between PDGFR and CLK3 (RMSD = 8.4) being lower than that between any other pair of proteins (RMSD ≤ 2.9) (Fig. 1A). In protein structures, the positions of key sites in the active centers of each kinase are highly conserved (Fig. 1B). Based on the above analysis results, the simultaneous inhibition of PDGFR, TNIK, and CLK by the biphenyl derivatives of the present application has a structural basis.

Using AutoDock Vina software, molecular docking was performed between biphenyl derivative molecules I-1 to I-18 and multiple target proteins. The binding energy and ligand efficiency of the docking results of each biphenyl derivative molecule with the target protein was calculated. The specific docking results are shown in Table 1: Columns 2-5 respectively display the binding energy of the compounds involved in this application with different target proteins, calculated by the docking software. The larger the absolute value, the stronger the binding ability of the small molecule ligand with the target protein. The predicted binding energy levels of the compounds involved in this application show that the binding energies of the compounds of this application are all much greater than the threshold value 3 set based on the target characteristics in this application. This result indicates that all biphenyl derivative molecules in this application have the ability to bind to the target proteins.

**Table 1. Energy analysis during the binding process between the compounds of this application and the target proteins**

| Compound | AD_vina_binding_energy (kcal/mol) | | | |
|---|---|---|---|---|
| | PDGFRα | TNIK | CLK4 | JNK1 |
| I-1 | -11.3 | -9.3 | -10.8 | -9.5 |
| I-2 | -11 | -9.6 | -10.8 | -9.2 |
| I-3 | -11.1 | -9.4 | -10.9 | -9.4 |
| I-4 | -11.7 | -9.8 | -10.7 | -8.6 |
| I-5 | -9.7 | -8.4 | -9.2 | -8.5 |
| I-6 | -10 | -9.1 | -9.7 | -8.6 |
| I-7 | -10.9 | -9.3 | -10.2 | -8.5 |
| I-8 | -10.7 | -9.2 | -9.9 | -9.4 |
| I-9 | -9.6 | -8.6 | -9.6 | -8.3 |
| I-10 | -10.7 | -9.1 | -10 | -9.1 |
| I-11 | -11.1 | -8.9 | -9.8 | -9.3 |
| I-12 | -11.1 | -9.5 | -10.3 | -9.2 |
| I-13 | -11 | -8.9 | -10.1 | -9 |
| I-14 | -10 | -8.7 | -9.5 | -8.1 |
| I-15 | -10 | -8.7 | -10 | -8.6 |
| I-16 | -10.1 | -8.8 | -9.7 | -8.7 |
| I-17 | -11.1 | -9.5 | -10.2 | -9.1 |
| I-18 | -10.9 | -9.3 | -10.2 | -9.1 |

Furthermore, molecular dynamics simulations were conducted on the biphenyl derivatives in this application. Molecular dynamics simulation is a computational method, which simulates and predicts the behavior of molecular systems through numerical integration based on Newton's mechanical principles and intermolecular interaction force fields. Molecular dynamics simulations can provide detailed information about protein structure, dynamics, and function, helping to understand the conformational changes of proteins, the characteristics of binding sites, and interactions with small molecules. By simulating molecular interactions, potential drug molecules can be screened, their interaction modes with proteins can be predicted, and their binding abilities can be evaluated. Therefore, this application tests the chemical basis of biphenyl derivative binding through molecular dynamics simulations; The principles and methods can be found in "Molecular Dynamics Simulations of Protein-Drug Complexes: A Computational Protocol for Investigating the Interactions of Small-Molecule Therapeutics with Biological Targets and Biosensors" (Hadden JA and Perilla JR, Methods Mol Biol. 2018; 1762:245-270).

The stability of each complex system was studied using Amber molecular dynamics simulation software. In the molecular dynamic simulation experiments, the ff14SB and Gaff force fields were employed for receptor proteins and ligand small molecules, respectively. The input files were prepared using the Antechamber and Leap programs of the Amber toolkit, followed by a 100 ns simulation calculation. The simulated system was prepared by introducing 0.15 M Na⁺ and Cl⁻ ions, along with a TIP3P water box with a side length of 15 Å, to mimic physiological salt concentration and neutralize the entire system. The shaking algorithm was used to constrain the bond lengths of all hydrogen bonds, and the particle-grid Ewald method was employed to calculate long-range electrostatic interactions. The system underwent 10,000 steps of minimization, followed by 10,000 steps of water balance. The system temperature was gradually equilibrated to 300K over 5000 steps from 200K. After equilibration, the prepared complex system was subjected to molecular dynamics simulation at a constant temperature of 300K and 1 atm.

The molecular dynamics simulation results indicate that during the 100 ns simulation process, the complex structures of each small molecule with the protein target gradually reached a stable state (Figs. 2-10, Series A). The binding energies between each small molecule and the protein target calculated using the MMGPSA program of Amber tool are presented in Table 1. Analysis of the energy contribution of amino acid sites on each protein target to the binding of ligand small molecules revealed that I-1, I-4, and I-12 share similar binding sites within the same target (Figs. 2-10, Series B); However, the energy contributions of the same amino acid site to the binding of different small molecules vary. For example, the energy contribution of LYS43 in TNIK to the binding of I-1, I-4, and I-12 are -0.39 kJ/mol, -2.36 kJ/mol, and -0.58 kJ/mol, respectively, which may be one of the reasons for the differences in sensitivity of TNIK to the three. Analysis of the complex structures stabilized by molecular dynamics simulation revealed that each small molecule was stably bound to the active catalytic center of the kinase target, while the amino acid sites involved in small molecule binding (with binding energy contributions less than -0.5 kJ/mol) mainly interacted directly with the small molecules through hydrogen bonds and hydrophobic interactions (Figs. 2-10, Series C).

As shown in Figs. 2-4, the A series of figures respectively represent the RMSD (Root Mean Square Deviation) changes of the complexes of TNIK with I-1, I-4, and I-12 over the simulation time during the simulation process. The results indicate that TNIK can bind to I-1, I-4, and I-12, and the formed complex structures are in a stable binding state. The B series of figures respectively show the energy decomposition diagrams of the interactions between each amino acid site of TNIK and I-1, I-4, and I-12. The x-axis represents the amino acid sequence of TNIK, and the y-axis represents the energy contribution of each amino acid site to ligand binding. The names of amino acid sites with a ligand binding energy contribution less than -0.5 kJ/mol are marked. This result suggests that the binding sites of TNIK with I-1, I-4, and I-12 are similar. Furthermore, the C series of figures respectively represent the complex structures of TNIK with I-1, I-4, and I-12 stabilized by molecular dynamics simulation. Proteins are displayed as cartoon models, with the colour of each site indicating its energy contribution to ligand binding. Small molecules and the main or side chains of the residues interacting with them are displayed as stick models, and hydrogen bond interactions are indicated by long dashed lines, while hydrophobic interactions are indicated by short dashed lines. This result demonstrates that the active catalytic centers of the kinase targets bound by TNIK with I-1, I-4, and I-12, as well as the chemical basis of their interactions, are clear; that is, the basis for the formation of chemical bonds is consistent.

As shown in Figs. 5-7, the A series of figures represent the RMSD (Root Mean Square Deviation) changes of the complexes of PDGFRα with I-1, I-4, and I-12 over the simulation time during the simulation process. The results indicate that PDGFRα can bind to I-1, I-4, and I-12, and the formed complex structures are in a stable binding state. The B series of figures respectively show the energy decomposition diagrams of the interactions between each amino acid site of PDGFRα and I-1, I-4, and I-12. The x-axis represents the amino acid sequence of PDGFRα, and the y-axis represents the energy contribution of each amino acid site to ligand binding. The names of amino acid sites with a ligand binding energy contribution less than -0.5 kJ/mol are marked. This result suggests that the binding sites of PDGFRα with I-1, I-4, and I-12 are similar. Furthermore, the C series of figures respectively represent the complex structures of PDGFRα with I-1, I-4, and I-12 stabilized by molecular dynamics simulation. Proteins are displayed as cartoon models, with the colour of each site indicating its energy contribution to ligand binding. Small molecules and the main chains or side chains of the residues interacting with them are displayed as stick models, and hydrogen bond interactions are indicated by long dashed lines, while hydrophobic interactions are indicated by short dashed lines. This result demonstrates that the active catalytic centers of the kinase targets bound by PDGFRα with I-1, I-4, and I-12, as well as the chemical basis of their interactions, are clear; that is, the basis for the formation of chemical bonds is consistent.

As shown in Figs. 8-10, the A series of graphs respectively represent the changes in RMSD (Root Mean Square Deviation) of the complexes of CLK4 with I-1, I-4, and I-12 over simulation time during the simulation process. The results indicate that CLK4 can bind to I-1, I-4, and I-12, and the formed complex structures are in a stable binding state. The B series of graphs respectively represent the energy decomposition diagrams of the interactions between each amino acid site of CLK4 and I-1, I-4, and I-12. The x-axis represents the amino acid sequence of CLK4, and the y-axis represents the energy contribution of each amino acid site to ligand binding. The names of amino acid sites with a ligand binding energy contribution less than -0.5 kJ/mol are marked. This result indicates that the binding sites of CLK4 with I-1, I-4, and I-12 are similar. Furthermore, the C series of graphs respectively represent the complex structures of CLK4 with I-1, I-4, and I-12 stabilized by molecular dynamics simulation. Proteins are displayed as cartoon models, with the colour of each site indicating its energy contribution to ligand binding. Small molecules and the main chains or side chains of the residues interacting with them are displayed as stick models, and hydrogen bond interactions are indicated by long dashed lines, while hydrophobic interactions are indicated by short dashed lines. This result demonstrates that the active catalytic centers of the kinase targets bound by CLK4 with I-1, I-4, and I-12, as well as the chemical basis of their interactions, are clear; that is, the basis for the formation of chemical bonds is consistent.

As shown in Figs. 11-12, the A series of figures respectively represent the RMSD (Root Mean Square Deviation) changes of the complexes of JNK1 with I-1 and I-4 over the simulation time during the simulation process. The results indicate that JNK1 can bind to both I-1 and I-4, and the formed complex structures are in a stable binding state. The B series of figures respectively represent the energy decomposition diagrams of the interactions between each amino acid site of JNK1 and I-1 and I-4. The x-axis represents the amino acid sequence of JNK1, and the y-axis represents the energy contribution of each amino acid site to ligand binding. The names of amino acid sites with a ligand binding energy contribution less than -0.5 kJ/mol are marked. This result suggests that the binding sites of JNK1 with I-1 and I-4 are similar. Furthermore, the C series of figures respectively represent the complex structures formed by JNK1 with I-1 and I-4 stabilized by molecular dynamics simulation. Proteins are displayed as cartoon models, with the colour of each site indicating its energy contribution to ligand binding. Small molecules and the main chains or side chains of the residues interacting with them are displayed as stick models, and hydrogen bond interactions are indicated by long dashed lines, while hydrophobic interactions are indicated by short dashed lines. This result demonstrates that the active catalytic centers of the kinase targets bound by JNK1 with I-1, and I-4, as well as the chemical basis of their interactions, are clear; that is, the basis for the formation of chemical bonds is consistent.

In summary, the biphenyl derivatives in this application possess a structure capable of binding to a series of kinase targets and have a chemical basis for stable binding.

### Example 2: Biochemical screening of biphenyl derivatives and target kinases

Based on the results from Example 1, in vitro kinase profile screening was conducted on the biphenyl derivatives in this application to further confirm the actual functional interactions between these derivatives and target kinases. Through the screening of inhibitory effects on activity among 330 kinases, during the kinase activity assay, the kinase (15-50 nM, 2.5 µL, prepared in assay buffer) was first mixed with biphenyl derivative I-1 (10 mM, 25 nL, prepared in DMSO) and incubated at 25 °C for 10 minutes. Subsequently, a mixture of kinase peptide substrate (0.2 mg/ml; supplier: GenScript) and ATP (10-60 µM; supplier: Promega; product number: V915B) (total 2.5 µL, prepared in assay buffer) was added to the mixed system, and the reaction was allowed to proceed at 25 °C for 60-120 minutes. Finally, the reaction products were quantitatively detected using the HTRF or ADP-Glo method. The composition of the test buffer is as follows: 50 mM HEPES (supplier: Thermo Fisher; product number: 15630080), 10 mM MgCl₂ (supplier: Sigma; product number: M1028), 0.01% Brij35 (supplier: Millipore; product number: 1018940100), 1 mM EGTA (supplier: Sigma; product number: E3889), and 2 mM DTT (supplier: MCE; product number: HY-15917). During the kinase activity assay, the consumption of each substrate was less than 10%. Each kinase activity assay was set up with two technical replicates. The results indicated that biphenyl derivative I-1 could exert significant inhibitory effects on multiple target kinases (Figs. 13-14). This result further confirms the calculations and predictions from Example 1.

### Example 3: IC₅₀ screening of biphenyl derivatives against target kinases

To further evaluate the binding ability of biphenyl derivatives to target kinases, IC50 was used to detect the inhibitory effect of biphenyl derivatives on target kinases. In this experiment, IC₅₀ represents the concentration of drug or inhibitor required to inhibit the activity of a specified kinase by half. In pharmacology, it is used to characterize the antagonistic ability of antagonists in in vitro experiments. The specific operational steps for IC₅₀ are referred to in "Assessing the Inhibitory Potential of Kinase Inhibitors In Vitro: Major Pitfalls and Suggestions for Improving Comparability of Data Using CK1 Inhibitors as an Example" (Roth A, et al., Molecules. 2021 Aug 12; 26(16):4898).

First, prepare 2 × ATP and substrate mixture solution and 2 × TNIK and MgCl₂ mixture solution using test buffer, respectively. Then, transfer 40 nL of small molecules and 2 µL of 2 × TNIK and MgCl₂ mixture solution to a 384-well plate. After mixing at room temperature, incubate for 10 minutes. Next, transfer 2 µL of 2 × ATP and substrate mixture solution to the 384-well plate, and react at room temperature for 60 minutes. Add 4 µL of ADP-Glo reagent (supplier: Promega; product number: V9103) to the reaction system, and incubate at room temperature for 40 minutes. Finally, add 8 µL of kinase detection reagent (supplier: Promega; product number: V9103) to the reaction system, incubate at room temperature for 40 minutes, and read the fluorescence signal using a microplate reader. The concentration gradients of biphenyl derivatives I-1, I-4, I-12 are set at 50000 µM, 12500 µM, 3125 µM, 781.3 µM, 195.3 µM, 48.83 µM, 12.21 µM, 3.05 µM, 0.76 µM, and 0.19 µM.

The calculation formula for inhibition rate (%) is: inhibition rate (%) = 100% - (small molecule reading - positive control reading) / (negative control reading - positive control reading) * 100%. By substituting the inhibition rate (%) and the Log value of inhibitor concentration into the nonlinear regression equation, the IC₅₀ of the small molecule can be calculated as: Y = Bottom + (Top - Bottom) / (1 + 10^((LogIC₅₀ - X) * hillslope)), where X is the Log value of inhibitor concentration and Y is the inhibition rate (%). The results are shown in Fig. 15. Biphenyl derivatives I-1, I-4, and I-12 all exhibit binding ability to the target kinase and demonstrate significant antagonistic activity against the target kinase.

Based on the results of Example 2 and Example 3, it is indicated that all biphenyl derivatives are capable of binding to the target kinase.

### Example 4: Biphenyl derivatives can induce the simultaneous expression of the reprogramming core genes Oct4, Lin28A, and c-Myc

CHIR99021, as a widely used WNT pathway regulatory compound, plays a very significant role in maintaining pluripotency in mammalian embryos (Meek et al., STEM CELLS. 2007, 31, 10, p. 2104-2115), is considered as a potential reprogramming inducer. To further verify whether biphenyl derivatives can initiate cell reprogramming, CHIR99021 was also used in control experiments in this application.

Human mesenchymal cells were cultured in T25 flasks, seeded at a density of 4 × 10⁵ cells per flask, and cultured in serum-free Dulbecco's Modified Eagle Medium (DMEM-F12 medium) supplemented with 20µM of the aforementioned biphenyl derivatives. The culture conditions were maintained at 37°C with 5% carbon dioxide. On day 3, total RNA was extracted using the RNeasy Mini or Micro Kit (QIAGEN), and 1 mg of RNA was used to synthesize cDNA with the SuperScript III First-Strand Synthesis System (Invitrogen). SYBR Premix Ex Taq (TaKaRa) and the Thermal Cycler Dice Real Time System (TaKaRa) were used for labeling and reaction in Quantitative PCR, with beta-Actin serving as an internal control. All data were analyzed using the delta-Ct method. Each experiment was conducted in triplicate, and variance analysis was conducted. The primer sequences for identifying the coding genes of different cell markers are shown in Table 2. The results are shown in Fig. 16. Compared to the control group (CK) without the use of biphenyl derivative small molecules and the control group with the addition of CHIR99021 alone, only the biphenyl derivatives can simultaneously induce the expression of Oct4, Lin28A, and c-Myc. Therefore, the solitary use of biphenyl derivatives can significantly increase the expression of multiple reprogramming core genes. The simultaneous expression of the reprogramming core genes Oct4, Lin28A, and c-Myc is a necessary condition for the reprogramming of various somatic cells.

**Table 2. QPCR primer sequences for compound effect genes**

| sequence name | Primer Name | Primer sequence |
|---|---|---|
| Seq_1 | Oct4-F | CCATGCATTCAAACTGAGGT |
| Seq_2 | Oct4-R | CCTTTGTGTTCCCAATTCCTT |
| Seq_3 | c-Myc-F | CTGAGGAGGAACAAGAAGATGA G |
| Seq_4 | c-Myc-R | TGTGAGGAGGTTTGCTGTG |
| Seq_5 | Lin28A-F | TGTCTTTGTGCACCAGAGTAAG |
| Seq_6 | Lin28A-R | GACCCTTGGCTGACTTCTTAAA |
| Seq_7 | βActin-F | GGCCGAGGACTTTGATTGCACA |
| Seq_8 | βActin-R | GGGCACGAAGGCTCATCATTCAA |

The technical features described in the above examples can be combined in any manner. For the sake of brevity, not all possible combinations of the technical features in the above examples have been explicitly described. However, as long as the combination of these technical features does not result in a contradiction, it should be regarded as falling within the scope disclosed in this specification.

The above examples merely illustrate several embodiments of the present application in a specific and detailed manner, and should not be construed as limiting the scope of the invention. It should be noted that those skilled in the art, within the framework of the present application, may make various modifications and improvements, all of which are considered to fall within the protection scope of the present application. Therefore, the protection scope of the present patent application shall be defined by the appended claims, with the description and drawings serving to explain the content of the claims.

## Claims

1. A biphenyl derivative, wherein the biphenyl derivative has a structure represented by Formula I, or a pharmaceutically acceptable salt, ester, amide, solvate, active metabolite, polymorph, isotopic label, isomer, or prodrug thereof; 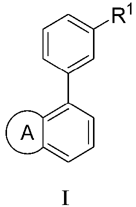
Wherein the A ring is a five-membered ring that is substituted with methyl or amino, or is unsubstituted, and contains one or two nitrogen atoms among its ring atoms;
R¹ is a hydrogen bond donor or acceptor, and its structure contains one or more of amino, imino, hydroxyl, and ether linkage.

2. The biphenyl derivative according to claim 1, wherein R¹ is selected from one of the following structures: 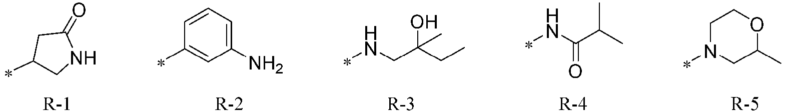 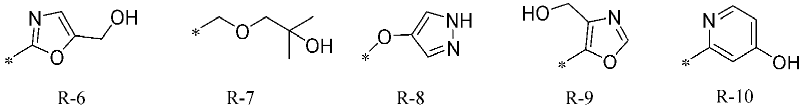 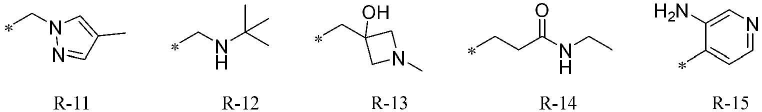 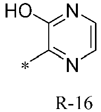 Wherein, "*" represents a point of attachment.

3. The biphenyl derivative according to claim 2, wherein R¹ is selected from one of the following structures: 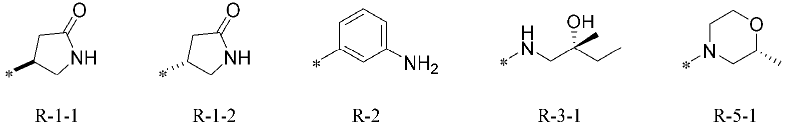 Wherein, "*" represents a point of attachment.

4. The biphenyl derivative according to claim 1, wherein the A ring is selected from one of the following structures: 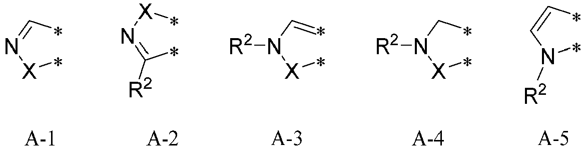
Wherein, X is CR³R⁴ or NR⁵, and R²-R⁵ are independently selected from -H, -CH₃, or -NH₂;
"*" represents a point of attachment.

5. The biphenyl derivative according to claim 3, wherein the A ring is selected from one of the following structures: 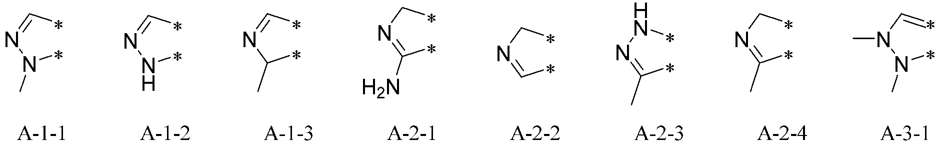 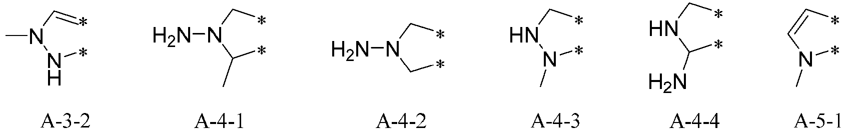 Wherein, "*" represents a point of attachment.

6. The biphenyl derivative according to claim 3, wherein the A ring is selected from one of the following structures: 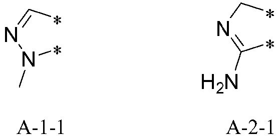 Wherein, "*" represents a point of attachment.

7. The biphenyl derivative according to claim 1, wherein the biphenyl derivative has a structure represented by any one of Formulas I-1 to I-18, or a pharmaceutically acceptable salt, ester, amide, solvate, active metabolite, polymorph, isotopic label, isomer, or prodrug thereof;

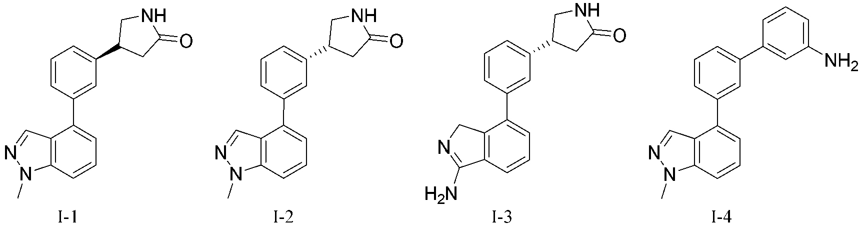

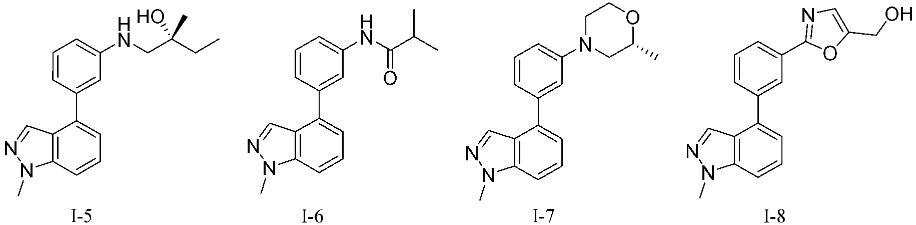

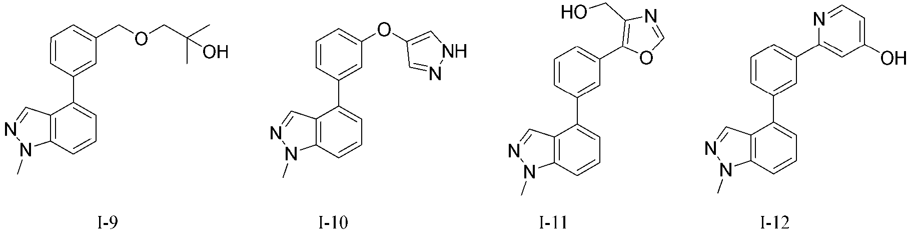

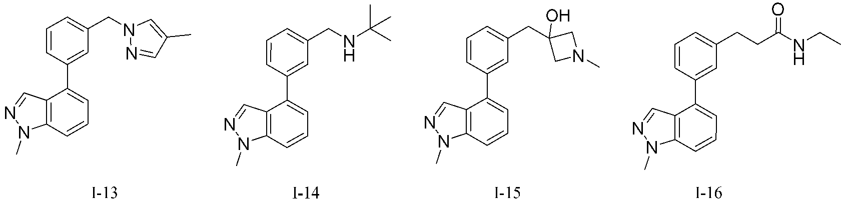

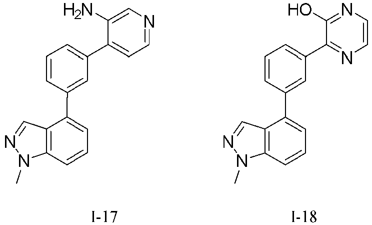

8. A pharmaceutical composition comprising the biphenyl derivative according to any one of claims 1 to 7, and at least one pharmaceutically acceptable carrier.

9. Use of the biphenyl derivative according to any one of claims 1 to 7, or the pharmaceutical composition according to claim 8, in cell reprogramming.

10. A method for cell reprogramming, comprising the following steps:
Contacting cells with the biphenyl derivative according to any one of claims 1 to 7 or the pharmaceutical composition according to claim 8.

11. The method according to claim 10, wherein the concentration of the biphenyl derivative during the contacting step is 1 µM to 50 µM.

12. Use of the biphenyl derivative according to any one of claims 1 to 7 or the pharmaceutical composition according to claim 8 in kinase inhibition, wherein the kinase comprises one or more of the TK kinase family, STE kinase family, and CMGC kinase family.

13. The use according to claim 12, wherein the kinase comprises one or more of PDGFRα, TNIK, CLK4, and JNK1.

14. A method for inhibiting kinase, comprising the following steps:
Contacting a kinase with the biphenyl derivative according to any one of claims 1 to 7 or the pharmaceutical composition according to claim 8;
Wherein the kinase comprises one or more of the TK kinase family, STE kinase family, and CMGC kinase family.

15. The method according to claim 14, wherein the kinase comprises one or more of PDGFRα, TNIK, CLK4, and JNK1.
